# EUROPÄISCHE PATENTSCHRIFT

(11) **EP 4 547 130 B1**
(45) Veröffentlichungstag und Bekanntmachung des Hinweises auf die Patenterteilung: **27.05.2026**
(21) Anmeldenummer: 23737932.6
(22) Anmeldetag: 29.06.2023
(51) Int. Cl.: A61B 17/29, A61B 90/00

(54) **CHIRURGISCHES INSTRUMENTENSYSTEM, CHIRURGISCHES INSTRUMENT UND VERFAHREN ZUR MONTAGE UND DEMONTAGE DES CHIRURGISCHEN INSTRUMENTS**
SURGICAL INSTRUMENT SYSTEM, SURGICAL INSTRUMENT, AND METHOD FOR ASSEMBLING AND DISASSEMBLING THE SURGICAL INSTRUMENT
SYSTÈME D'INSTRUMENT CHIRURGICAL, INSTRUMENT CHIRURGICAL ET PROCÉDÉ D'ASSEMBLAGE ET DE DÉSASSEMBLAGE D'INSTRUMENT CHIRURGICAL

(30) Priorität: 30.06.2022 DE 102022116379
(43) Veröffentlichungstag der Anmeldung: 07.05.2025
(73) Patentinhaber: Karl Storz SE & Co. KG, 78532 Tuttlingen (DE)
(72) Erfinder: SCHNEIDER, Janosz, 78532 Tuttlingen (DE); KÄRCHER, Daniel, 78532 Tuttlingen (DE); MERZ, Robin, 78532 Tuttlingen (DE); SCHNEIDER, Sven, 78532 Tuttlingen (DE); UNGER, Tobias, 78532 Tuttlingen (DE); LÄNGLE, Dominik, 78532 Tuttlingen (DE); PÖNISCH, Judith, 78532 Tuttlingen (DE)
(86) Internationale Anmeldenummer: PCT/EP2023/067774
(87) Internationale Veröffentlichungsnummer: WO 2024/003218

(56) Entgegenhaltungen:
- CN-A- 109 276 292
- CN-U- 211 325 745
- DE-A1- 102007 021 658
- US-A- 5 015 252
- US-A1- 2019 008 547
- US-B2- 10 582 944

## Beschreibung

Die Erfindung betrifft ein chirurgisches Instrumentensystem, ein daraus reversibel zusammenbaubares und zerlegbares chirurgisches Instrument und ein entsprechendes Verfahren zur Montage und Demontage eines chirurgischen Instruments.

Aus dem Stand der Technik ist bekannt, dass chirurgische Instrumente mit Werkzeugen ausgerüstet sein können, die durch Betätigung einer Handhabe über ein hin und her bewegbares Übertragungselement bewegt werden können. Dieses häufig stabförmige Übertragungselement erstreckt sich durch einen langerstreckten zylindrischen Schaft, an dessen distalem Ende das mit dem Übertragungselement verbundene Werkzeug angeordnet ist. Am proximalen Ende des Schafts ist ein Handgriff angeordnet, der mit dem Übertragungselement in Wirkverbindung steht.

Hierbei ist bekannt, dass modulare chirurgische Instrumentensysteme es dem Anwender ermöglichen, ein von ihm gewünschtes chirurgisches Instrument jederzeit individuell neu zusammenzustellen, das auch wieder einfach in die Instrumentenkomponenten zerlegt, gereinigt und zumindest teilweise wiederverwendet werden kann. Ein modulares System für chirurgische Instrumente kann beispielsweise drei Baukästen mit den Instrumentenkomponenten Handgriff, Schaft und Werkzeugspitze aufweisen. Die Werkzeugspitzen werden häufig in Verbindung mit einem jeweiligen stabförmigen Übertragungselement bereitgestellt. Die zerlegbare Bauweise gestaltet den Austausch defekter Teile einfach und ermöglicht durch autoklavierbare Komponenten zumindest teilweise eine Wiederverwendung. Die im modularen System auswählbaren Handgriffe, Schäfte und Werkzeugspitzen können sich in ihren Anwendungsgebieten, Materialien und/oder Größen unterscheiden. Beispielsweise können die Handgriffe unterschiedliche Griffteildesigns aufweisen und beispielsweise mit diversen unterschiedlichen Funktionselementen, beispielsweise Rasten, Spülanschlüssen und Betätigungselementen zur Schaftrotation ausgerüstet sein. Für elektrische Hochfrequenzanwendungen (Erzeugung von Wärme durch hochfrequenten Wechselstrom zur Entfernung oder zum Schneiden von Gewebe bzw. Blutungsstillung durch Gefäßverschluss) kann das System ferner isolierte Versionen von Handgriffen und Schäften umfassen.

Um beim Zerlegen eines modularen chirurgischen Instruments den Schaft vom Handgriff händisch ohne zusätzliches Montagewerkzeug trennen zu können, sind Handgriffe mit einer Mechanik bekannt, die per Knopfdruck den Handgriff vom Schaft löst, wie beispielsweise in DE 10 2012 007 650 A1 offenbart ist.

Aus EP 0 546 209 B1, die eine endoskopische Zange zur Anwendung von Hochfrequenzströmen betrifft, ist bekannt, den Schaft durch eine Rändelschraube in einem feststehenden Griffteil des Griffs lösbar festzuklemmen, sodass kein zusätzliches Montagewerkzeug bei der Demontage des modularen chirurgischen Instruments erforderlich ist.

Ferner werden zum lösbaren Verbinden von rohrförmigen Komponenten eines modularen chirurgischen Systems häufig Bajonettverbindungen, wie beispielsweise in DE 197 07 373 C1 oder EP 0 688 187 B1 offenbart sind, oder Rastverbindungen eingesetzt, bei denen radial komprimierbare elastische Rastarme aus einem geschlitzten Rohrende mit nach außen weisenden Vorsprüngen in entsprechende Ausnehmungen in einer Rohraufnahme eingreifen können, wie etwa aus EP 0 712 608 A2 und US 5,947,996 A bekannt sind.

Auch eine Kombination von Bajonett- und Rastverschluss ist bekannt, beispielsweise aus DE 10 2010 048516 B4, wobei der in der Bajonett-Führungsbahn geführte Bajonett-Führungskörper zugleich einen Rastkörper bildet, der in Bajonett-Verschlussstellung mit einem Klemmmittel in Eingriff kommt, das in die Bajonett-Führungsbahn ragend am freien Ende einer federnden Lasche angeordnet ist.

DE 10 2007 021658 A1 offenbart ein chirurgisches Instrumentensystem mit einem zerlegbaren chirurgischen Instrument.

Dass die Komponenten eines modular zusammengebauten chirurgischen Instruments zerlegbar sind, indem die jeweiligen Komponenten gleichzeitig gegriffen und gegeneinander verdreht und/oder auseinandergezogen werden, ggf. unter gleichzeitiger Betätigung eines Elements zum Lösen eines Rasteingriffs, ermöglicht dem Anwender das Zerlegen ohne zusätzliches Montagewerkzeug. Allerdings sind durch die Vorgabe der werkzeuglosen Zerlegbarkeit die Verbindungsmöglichkeiten und die Kräfte, die zur Zerlegung aufgebracht werden können, beschränkt.

Ausgehend von diesem Stand der Technik ist es Aufgabe der vorliegenden Erfindung, ein verbessertes Instrumentensystem für ein verbessertes chirurgisches Instrument bereitzustellen.

Diese Aufgabe wird durch ein Instrumentensystem mit den Merkmalen des Anspruchs 1 und durch ein zerlegbares chirurgisches Instrument mit den Merkmalen des Anspruchs 11 gelöst. Die weitere Aufgabe der vereinfachten Montage und Demontage eines chirurgischen Instruments wird durch das Verfahren mit den Merkmalen des Anspruchs 12 gelöst.

Bevorzugte Ausführungsformen sind in den Unteransprüchen ausgeführt.

Eine erste Ausführungsform des erfindungsgemäßen chirurgischen Instrumentensystems, das als Instrumentenkomponenten zumindest eine Werkzeugspitze, zumindest einen Schaft und zumindest einen Handgriff zum modularen Zusammenbau zumindest eines chirurgischen Instruments aufweist, bezieht sich darauf, dass die Werkzeugspitze und ein distales Ende des Schafts als distale Verbindungspartner ausgebildet sind, die in lösbarem Eingriff verbindbar sind, und ein proximales Ende des Schafts und der Handgriff als proximale Verbindungspartner ausgebildet sind, die ebenfalls in lösbarem Eingriff verbindbar sind. Erfindungsgemäß ist vorgesehen, dass zumindest eine der Instrumentenkomponenten ein integriertes Montagewerkzeug aufweist, das zur Zusammenwirkung mit zumindest einem der distalen Verbindungspartner oder einem der proximalen Verbindungspartner ausgebildet ist, um den Eingriff des zusammenwirkenden distalen Verbindungspartners oder den Eingriff des zusammenwirkenden proximalen Verbindungspartners zu lösen oder zu fügen. Hierbei soll umfasst sein, dass das Montagewerkzeug gegebenenfalls auch zur Zusammenwirkung mit einem der distalen Verbindungspartner und mit einem der proximalen Verbindungspartner ausgebildet ist, um sowohl den Eingriff des zusammenwirkenden distalen Verbindungspartners als auch den Eingriff des zusammenwirkenden proximalen Verbindungspartners lösen oder fügen zu können.

Durch den Einsatz eines Montagewerkzeugs, das infolge der Integration in eine Instrumentenkomponente bei Bedarf immer zur Verfügung steht, kann die mechanische Stabilität der jeweiligen Verbindung erhöht werden und - je nach Ausführungsform - etwaiges Spiel der Instrumentenkomponenten reduziert werden. Ferner wird der Vorgang der Zerlegung vereinfacht.

Vorteilhaft ist weiter, dass das erfindungsgemäße chirurgische Instrumentensystem modular gestaltet und zusammengebaut werden kann.

Als erfindungsgemäßes chirurgisches Instrumentensystem zum Zusammenbau eines chirurgischen Instruments wird bereits ein Set aus einer Werkzeugspitze, einem Schaft und einem Handgriff betrachtet, wenn eine der Instrumentenkomponenten ein integriertes Montagewerkzeug aufweist, mit dem die Verbindung zwischen den anderen beiden Instrumentenkomponenten gelöst bzw. gefügt werden kann. Üblicherweise enthält ein chirurgisches Instrumentensystem als Baukastensystem mehrere Varianten der drei Instrumentenkomponenten Werkzeugspitze, Schaft und Handgriff, die zur Bildung eines individuellen chirurgischen Instruments beliebig nahezu beliebig kombiniert werden können. Die Anzahl der Werkzeugspitzen, Schäfte und Handgriffe in einem chirurgischen Instrumentensystem könnte gleich sein, sodass die Instrumentenkomponenten in mehrere Sets aufgeteilt werden können, ohne dass eine Instrumentenkomponente übrigbleibt. Alternativ können sich die Anzahlen der Werkzeugspitzen, Schäfte und Handgriffe in einem chirurgischen Instrumentensystem unterscheiden, vorausgesetzt, dass zumindest ein vollständiges Set der Instrumentenkomponenten, vorzugsweise mehrere vollständige Sets enthalten sind. Daher kann ein erfindungsgemäßes chirurgisches Instrumentensystem mehrere Sets der Instrumentenkomponenten und weitere Instrumentenkomponenten aufweisen, die kein vollständiges Set ergeben. Beispielsweise kann ein chirurgisches Instrumentensystem eine Anzahl an Werkzeugspitzen aufweisen, die größer ist als eine Anzahl an Handgriffen, die wiederum größer ist als die Anzahl an Schäften, sodass die Anzahl der enthaltenen Sets der Anzahl der Schäfte entspricht und das Instrumentensystem zusätzlich weitere Handgriffe und Werkzeugspitzen als Instrumentenkomponenten aufweist.

Die Instrumentenkomponente "Werkzeugspitze" umfasst hierbei ein chirurgisches Werkzeug, das beispielsweise eine Greif- oder Präparierzange, ein Stanz- oder Scherenwerkzeug sein kann, eine damit verbundene Betätigungsmechanik für das Werkzeug und einen Werkzeugeinsatz, der zur Halterung des Werkzeugs, zur Aufnahme der Betätigungsmechanik und zur Verbindung mit einem distalen Ende des Schafts ausgebildet sein kann. Ferner wird hier der Werkzeugspitze das stabförmige Übertragungselement zugeordnet, das distalseitig mit der Betätigungsmechanik verbunden ist. Die Instrumentenkomponente "Schaft" bezieht sich auf einen langerstreckten zylindrischen Schaft, an dessen distalem Ende die Werkzeugspitze angeordnet ist, sodass sich das der Werkzeugspitze zugeordnete Übertragungselement durch den Schaft erstreckt. Das proximale Ende des Schafts, der ggf. ein proximales Endstück aufweisen kann, ist zur Verbindung mit der Instrumentenkomponente Handgriff vorgesehen.

Eine weitere Ausführungsform des erfindungsgemäßen chirurgischen Instrumentensystems bezieht sich darauf, dass aus zumindest einem ersten Set der Instrumentenkomponenten zumindest ein erstes chirurgisches Instrument zusammenbaubar ist, das die Instrumentenkomponente mit dem integrierten Montagewerkzeug als einen der distalen Verbindungspartner oder als einen der proximalen Verbindungspartner aufweist. Dabei ist der Eingriff der Instrumentenkomponente, die das integrierte Montagewerkzeug aufweist, mit dem zugeordneten jeweiligen distalen oder proximalen Verbindungspartner manuell lösbar und fügbar. D. h., dass der Eingriff der proximalen Verbindungspartner manuell lösbar und fügbar ist, wenn die Instrumentenkomponente mit dem Montagewerkzeug einer der proximalen Verbindungspartner ist, wobei das Montagewerkzeug zur Zusammenwirkung mit einem der proximalen Verbindungspartner ausgebildet ist, und umgekehrt, dass der Eingriff der distalen Verbindungspartner manuell lösbar und fügbar ist, wenn die Instrumentenkomponente mit dem Montagewerkzeug einer der distalen Verbindungspartner ist, wobei das Montagewerkzeug zur Zusammenwirkung mit einem der distalen Verbindungspartner ausgebildet ist. Alternativ dazu kann aus zumindest einem Set der Instrumentenkomponenten zumindest ein erstes chirurgisches Instrument zusammenbaubar sein, bei dem das Montagewerkzeug manuell lösbar an der Instrumentenkomponente vorliegt und zur Zusammenwirkung mit einem der distalen Verbindungspartner und einem der proximalen Verbindungspartner ausgebildet ist, um sowohl den Eingriff des zusammenwirkenden distalen Verbindungspartners als auch den Eingriff des zusammenwirkenden proximalen Verbindungspartners zu lösen oder zu fügen.

Als "manuell lösbare" Verbindung wird eine Verbindung verstanden, die ohne Zuhilfenahme eines Werkzeugs mit den Händen gelöst sowie gefügt werden kann.

Eine Variante des erfindungsgemäßen chirurgischen Instrumentensystems, das mehrere erste Sets der Instrumentenkomponenten enthält, kann dabei vorsehen, dass aus jedem Set ein erstes chirurgisches Instrument zusammengebaut werden kann, bei dem die Instrumentenkomponente mit dem integrierten Montagewerkzeug in manuell lösbaren Eingriff mit ihrem jeweiligen Verbindungspartner bringbar ist, wobei das Montagewerkzeug zur Zusammenwirkung mit den beiden anderen Verbindungspartnern ausgebildet ist. Oder alternativ kann aus jedem Set ein erstes chirurgisches Instrument zusammengebaut werden, bei dem das Montagewerkzeug manuell lösbar an der Instrumentenkomponente vorliegt und zur Zusammenwirkung mit jeweils einem der distalen und proximalen Verbindungspartner ausgebildet ist.

Als "erstes" Set bzw. chirurgisches Instrument wird hierbei nicht eine bestimmte Stelle innerhalb einer Mengenangabe bezeichnet, sondern ein erster Typ eines Instrumentensets bzw. eines chirurgischen Instruments, das eine Instrumentenkomponente mit dem Montagewerkzeug aufweist, wobei entweder die Instrumentenkomponente mit dem Montagewerkzeug von ihrem zugeordneten Verbindungpartner oder das Montagewerkzeug von der Instrumentenkomponente manuell lösbar ist, sodass bereits ein einziges erstes Set ausreichend ist, um das erste chirurgische Instrument zusammenbauen und zerlegen zu können.

Alternativ oder zusätzlich zu einem ersten Set der Instrumentenkomponenten zum Zusammenbau eines ersten chirurgischen Instruments kann ein erfindungsgemäßes chirurgisches Instrumentensystem in einer weiteren Ausführungsform zumindest ein zweites Set der Instrumentenkomponenten aufweisen, aus denen ein zweites chirurgisches Instrument zusammenbaubar ist. Ein "zweites" chirurgisches Instrument bzw. Set bezieht sich auf einen von dem "ersten" chirurgischen Instrument bzw. Set abweichenden Typ, wobei entweder keine der Instrumentenkomponenten ein Montagewerkzeug aufweist oder das Montagewerkzeug nicht-lösbar an einer Instrumentenkomponente vorliegt, deren Eingriff mit dem zugeordneten Verbindungspartner nicht manuell lösbar ist. Im Unterschied zu einem ersten Set bzw. chirurgischen Instrument ist ein einziges zweites Set nicht ausreichend, um das zweite chirurgische Instrument zusammenbauen und zerlegen zu können. Ein erfindungsgemäßes chirurgisches Instrumentensystem mit einem solchen "zweiten" Set weist daher zum Zusammenbauen und Zerlegen eines "zweiten" chirurgischen Instruments zumindest eine weitere Instrumentenkomponente mit einem integrierten Montagewerkzeug bzw. ein entsprechendes Set mit der weiteren Instrumentenkomponente auf, durch die das Montagewerkzeug zum Zusammenbauen und Zerlegen des "zweiten" chirurgischen Instruments bereitgestellt wird. Diese weitere Instrumentenkomponente kann eine Werkzeugspitze oder ein Schaft oder ein Handgriff sein, wobei das in der weiteren Instrumentenkomponente integrierte Montagewerkzeug zur Zusammenwirkung mit jeweils einem der distalen Verbindungspartner und einem der proximalen Verbindungspartner des zweiten chirurgischen Instruments zum Lösen oder Fügen beider Eingriffe ausgebildet ist.

In einer Variante dieser Ausführungsform eines erfindungsgemäßen chirurgischen Instrumentensystems ist vorgesehen, dass das chirurgische Instrumentensystem zumindest ein erstes Set und ein zweites Set, d. h. zumindest zwei Werkzeugspitzen, zumindest zwei Schäfte und zumindest zwei Handgriffe als Instrumentenkomponenten aufweist, aus denen ein erstes chirurgische Instrument und ein zweites chirurgisches Instrument zusammenbaubar sind. Hierbei ist das zweite chirurgische Instrument ein werkzeugloses chirurgisches Instrument, bei dem keine Instrumentenkomponente ein Montagewerkzeug aufweist. Ferner entspricht die Instrumentenkomponente des ersten Sets, die das Montagewerkzeug aufweist, der in Bezug auf das zweite Set weiteren Instrumentenkomponente mit dem integrierten Montagewerkzeug, das folglich nicht nur zur Zusammenwirkung mit zumindest einem der distalen oder proximalen Verbindungspartner des ersten Sets bzw. ersten chirurgischen Instruments, sondern auch zur Zusammenwirkung mit jeweils einem der distalen und proximalen Verbindungspartner des zweiten Sets bzw. des zweiten werkzeuglosen chirurgischen Instruments ausgebildet ist. Mit dem Montagewerkzeug des ersten Sets bzw. des ersten chirurgischen Instruments kann folglich nicht nur das erste chirurgische Instrument zusammengebaut und zerlegt werden, sondern können auch die Eingriffe des jeweils zusammenwirkenden distalen und proximalen Verbindungspartners des zweiten werkzeuglosen chirurgischen Instruments gelöst oder gefügt werden.

In einer bevorzugte Ausführungsform eines erfindungsgemäßen chirurgischen Instrumentensystems, das zumindest ein erstes Set Instrumentenkomponenten aufweist, aus denen sich zumindest ein erstes chirurgisches Instrument zusammenbauen lässt, ist vorgesehen, dass die Instrumentenkomponente mit dem integrierten Montagewerkzeug der Handgriff ist, da dieser ausreichend Möglichkeiten bzw. Bauraum zur Integration eines Montagewerkzeugs bietet. Entsprechend ist der Handgriff für einen manuell lösbaren Eingriff mit dem proximalen Ende des Schafts ausgebildet, wobei das Montagewerkzeug zur Zusammenwirkung mit einem der distalen Verbindungspartner Werkzeugspitze oder distales Schaftende ausgebildet ist, um den entsprechenden Eingriff des zusammenwirkenden distalen Verbindungspartners zu lösen oder zu fügen.

Alternativ dazu und aus Bauraumgründen ggf. weniger bevorzugt kann die Instrumentenkomponente mit dem integrierten Montagewerkzeug die Werkzeugspitze sein, die dann für einen manuell lösbaren Eingriff mit dem distalen Ende des Schafts ausgebildet ist, um mit dem integrierten Montagewerkzeug, das zur Zusammenwirkung mit einem der proximalen Verbindungspartner Handgriff oder proximales Schaftende ausgebildet ist, den Eingriff des zusammenwirkenden distalen Verbindungspartners zu lösen oder zu fügen.

In einer weiteren Alternative, in der das Montagewerkzeug manuell lösbar an der Instrumentenkomponente angeordnet ist, kann diese wahlweise der Handgriff oder der Schaft oder die Werkzeugspitze sein, wobei das lösbare Montagewerkzeug zur Zusammenwirkung sowohl mit einem der distalen Verbindungspartner als auch mit einem der proximalen Verbindungspartner ausgebildet sein kann, um die Eingriffe des jeweils zusammenwirkenden distalen und proximalen Verbindungspartners zu lösen oder zu fügen.

Gemäß einer weiteren alternativen Ausführungsform eines erfindungsgemäßen chirurgischen Instrumentensystems, das kein erstes Set aufweist, kann das chirurgische Instrumentensystem zumindest zwei zweite Sets, also zumindest zwei Werkzeugspitzen, zumindest zwei Schäfte und zumindest zwei Handgriffe als Instrumentenkomponenten zum Zusammenbau von zumindest zwei zweiten chirurgischen Instrumenten aufweisen, bei denen weder die Eingriffe der distalen und proximalen Verbindungpartner noch die Anordnung des Montagewerkzeugs manuell lösbar ausgebildet sind und die daher hier als werkzeuglösbare chirurgische Instrumente bezeichnet werden. Dabei weist jedes zweite Set die in Bezug auf das jeweils andere zweite Set weitere Instrumentenkomponente mit dem integrierten Montagewerkzeug auf, das nicht lösbar an der Instrumentenkomponente angeordnet ist, deren Eingriff mit dem zugeordneten Verbindungpartner nicht manuell lösbar ist. Somit ist das Montagewerkzeug jedes zweiten werkzeuglösbaren chirurgischen Instruments zur Zusammenwirkung mit jeweils einem der distalen und einem der proximalen Verbindungspartner eines anderen zweiten chirurgischen Instruments ausgebildet, um den Eingriff des zusammenwirkenden distalen Verbindungspartners und den Eingriff des zusammenwirkenden distalen Verbindungspartners des jeweils anderen zweiten chirurgischen Instrument zu lösen oder zu fügen.

Für alle vorgenannten Varianten eines erfindungsgemäßen chirurgischen Instrumentensystems ist in einer weiteren Ausführungsform vorgesehen, dass das Montagewerkzeug ein Eingriffsprofil aufweist, das mit einem Mitnahmeprofil des zusammenwirkenden distalen oder proximalen Verbindungspartners korrespondiert. Das Mitnahmeprofil kann dabei an dem zusammenwirkenden distalen oder proximalen Verbindungspartner selbst vorliegen bzw. ausgebildet sein. Alternativ kann dem zusammenwirkenden distalen oder proximalen Verbindungspartner ein zusätzliches Verbindungselement zugeordnet sein, welches das Mitnahmeprofil aufweist, das mit dem Eingriffsprofil des Montagewerkzeugs korrespondiert, um die Zusammenwirkung bereitzustellen.

Weitere Ausführungsformen beziehen sich darauf, dass das Eingriffsprofil des Montagewerkzeugs in einem Funktionselement der Instrumentenkomponente ausgebildet sein kann, das eine von der Montagefunktion abweichende Basisfunktion hat. Beispiele für Funktionselemente mit abweichender Basisfunktion können Rasten, Spülanschlüsse, Betätigungssowie Gehäuseelemente umfassen, sind aber nicht darauf beschränkt. Alternativ kann das Montagewerkzeug durch ein Werkzeugelement bereitgestellt werden, das das Eingriffsprofil aufweist und das schwenkbar oder lösbar an der Instrumentenkomponente angeordnet oder in einer Aussparung der Instrumentenkomponente aufgenommen sein kann. Die lösbare Anordnung des Montagewerkzeugs ist dabei weniger bevorzugt, da bei mangelnder Sorgfalt das Risiko besteht, dass das Montagewerkzeug nach Benutzung verlegt werden oder verloren gehen kann. Daher ist ein lösbares Montagewerkzeug vorzugsweise auf die Variante eines ersten Sets der Instrumentenkomponenten zum Zusammenbau eines ersten chirurgischen Instruments beschränkt, bei dem die Eingriffe sowohl der distalen als auch der proximalen Verbindungspartner durch das Montagewerkzeug lösbar und fügbar ausgebildet sind.

So kann in einer beispielhaften Ausführungsform das Funktionselement der Instrumentenkomponente mit der von der Montagefunktion abweichenden Basisfunktion ein Drehknopf einer Rändelschraube sein, die zur manuellen Sicherung des proximalen Endes des Schafts im Handgriff angeordnet ist. In einer alternativen beispielhaften Ausführungsform kann die Aussparung ein an das Montagewerkzeug angepasstes Aufnahmefach sein, das in einem Griffgehäuse des Handgriffs ausgebildet ist.

Ein chirurgisches Instrumentensystem bezieht sich in weiteren erfindungsgemäßen Ausführungsformen darauf, dass das Eingriffsprofil entsprechend einem Schraubenschlüssel-Werkzeugprofil eines Maulschlüssels, Ringschlüssels, Rohrschlüssels oder Steckschlüssels ein Außen- oder Innenmehrkantprofil (z. B. Sechskant), Außen- oder Innenmehrrundprofil (z. B. Sechsrund) oder Außen- oder Innenvielzahnprofil sein kann. Alternativ kann das Eingriffsprofil einem Schraubenzieher-Werkzeugprofil für Schlitz- oder Kreuzschlitzschrauben entsprechen. Weitere Beispiele für Eingriffsprofile umfassen auch ein variierbares Greifprofil in Form einer Zange für diverse Mitnahmeprofile, die sich mit der Zange fassen lassen, oder ein individuell gestaltetes Werkzeugprofil, das von dem Schraubenschlüssel-Werkzeugprofil, dem Schraubenzieher-Werkzeugprofil und der Zange abweicht und auf ein individuell geformtes Mitnahmeprofil abgestimmt ist.

Ein ebenfalls erfindungsgemäßes zerlegbares chirurgisches Instrument weist in einer ersten Ausführungsform eine Werkzeugspitze, einen Schaft und einen Handgriff als Instrumentenkomponenten auf, wobei die Werkzeugspitze und ein distales Ende des Schafts als distale Verbindungspartner in lösbarem Eingriff stehen, und auch ein proximales Ende des Schafts und der Handgriff als proximale Verbindungspartner in lösbarem Eingriff stehen. Erfindungsgemäß ist das zerlegbare chirurgische Instrument aus den Instrumentenkomponenten eines erfindungsgemäßen chirurgischen Instrumentensystems zusammengebaut, bevorzugt aus den Instrumentenkomponenten eines ersten Sets. Das zerlegbare chirurgische Instrument weist eine erhöhte mechanische Stabilität mit reduziertem Spiel zwischen zumindest zwei Instrumentenkomponenten auf, die die distalen oder proximalen Verbindungspartner sind, deren Eingriff durch ein Montagewerkzeug lösbar ist, das an einer der Instrumentenkomponenten des chirurgischen Instrumentensystems, bevorzugt an einer Instrumentenkomponente des zerlegbaren chirurgischen Instruments selbst, integriert vorliegt.

Die Integration des Montagewerkzeugs in eine Instrumentenkomponente sichert vorteilhaft, dass das zur Montage und Demontage erforderliche Werkzeug bei Bedarf immer zur Hand ist und nicht verlegt oder verloren gehen kann. Damit wird vermieden, dass die Instrumente in einer von der vorgesehenen Weise abweichenden Art zerlegt oder montiert werden, was zu einer Beschädigung oder Beeinträchtigung am Instrument führen könnte.

Ein erfindungsgemäßes Verfahren zur Montage und Demontage eines erfindungsgemäßen chirurgischen Instruments, das aus den Instrumentenkomponenten eines erfindungsgemäßen chirurgischen Instrumentensystems zusammengebaut und zerlegt werden kann, umfasst in einer ersten Ausführungsform die Schritte
- Bereitstellen der Instrumentenkomponente mit dem Montagewerkzeug, das zum Zusammenwirken mit zumindest einem der distalen Verbindungspartner oder einem der proximalen Verbindungspartner ausgebildet ist, und
- unter Verwendung des Montagewerkzeugs bei der Montage des chirurgischen Instruments Fügen zumindest des Eingriffs der distalen Verbindungspartner oder des Eingriffs der proximalen Verbindungspartner und bei der Demontage des chirurgischen Instruments Lösen zumindest des Eingriffs der distalen Verbindungspartner oder des Eingriffs der proximalen Verbindungspartner.

Weitere erfindungsgemäße Ausführungsformen des Verfahrens unterscheiden sich in Abhängigkeit der jeweiligen Ausführungsform des erfindungsgemäßen chirurgischen Instrumentensystems, wobei die jeweiligen Verfahrensschritte der vorstehenden Beschreibung des erfindungsgemäßen chirurgischen Instrumentensystems bzw. der nachfolgenden detaillierten Beschreibung entnommen werden können.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine teilgeschnittene Seitenansicht eines zerlegbaren chirurgischen Instruments mit integriertem Montagewerkzeug nach einer erfindungsgemäßen Ausführungsform,
- **Fig. 2**: ein Seitenansicht eines chirurgischen Instrumentensystems nach einer erfindungsgemäßen Ausführungsform,
- **Fig. 3**: schematische Seitenansichten von erfindungsgemäßen chirurgischen Instrumenten mit unterschiedlichen Zerlegekonzepten a, b, c,
- **Fig. 4**: schematische Seitenansichten von erfindungsgemäßen chirurgischen Instrumentensystemen mit unterschiedlichen Zerlegekonzepten a, b,
- **Fig. 5**: eine perspektivische Detailansicht eines distalen Endabschnitts des Schafts im Eingriff mit der Werkzeugspitze und eines am Handgriff integrierten Montagewerkzeugs nach einer erfindungsgemäßen Ausführungsform,
- **Fig. 6**: eine schematische Seitenansicht eines am Handgriff integrierten Montagewerkzeugs nach einer weiteren erfindungsgemäßen Ausführungsform.

Die Erfindung betrifft ein modulares chirurgisches Instrumentensystem, das die Instrumentenkomponenten Handgriff, Schaft und Werkzeugspitze aufweist, sowie ein daraus zusammenbaubares chirurgisches Instrument. Ferner betrifft die Erfindung ein Verfahren zur Montage und Demontage eines chirurgischen Instruments aus den Instrumentenkomponenten eines erfindungsgemäßen modularen chirurgischen Instrumentensystems. Erfindungsgemäß ist ein Montagewerkzeug in eine der Instrumentenkomponenten des chirurgischen Instrumentensystems integriert, mit der sich zumindest eine Verbindung zweier Instrumentenkomponenten lösen und fügen lässt. Die als Montagewerkzeug bezeichnete Vorrichtung ist folglich nicht auf die Anwendung bei der Montage des chirurgischen Instruments zum Fügen der jeweiligen Verbindung beschränkt, sondern kann ebenso bei der Demontage des chirurgischen Instruments zum Lösen der entsprechenden Verbindung eingesetzt werden. Unter einem integrierten Montagewerkzeug wird sowohl ein Werkzeugelement wie z. B. ein Maulschlüssel verstanden, der schwenkbar oder lösbar in einer Aussparung einer Instrumentenkomponente aufgenommen ist, als auch eine Werkzeugfunktionalität, die integral an oder in einem bestehenden Funktionselement der Instrumentenkomponente des chirurgischen Instrumentensystems vorliegt. Das Funktionselement hat dabei üblicherweise eine von der Montage- und Demontagefunktion abweichenden Basisfunktion.

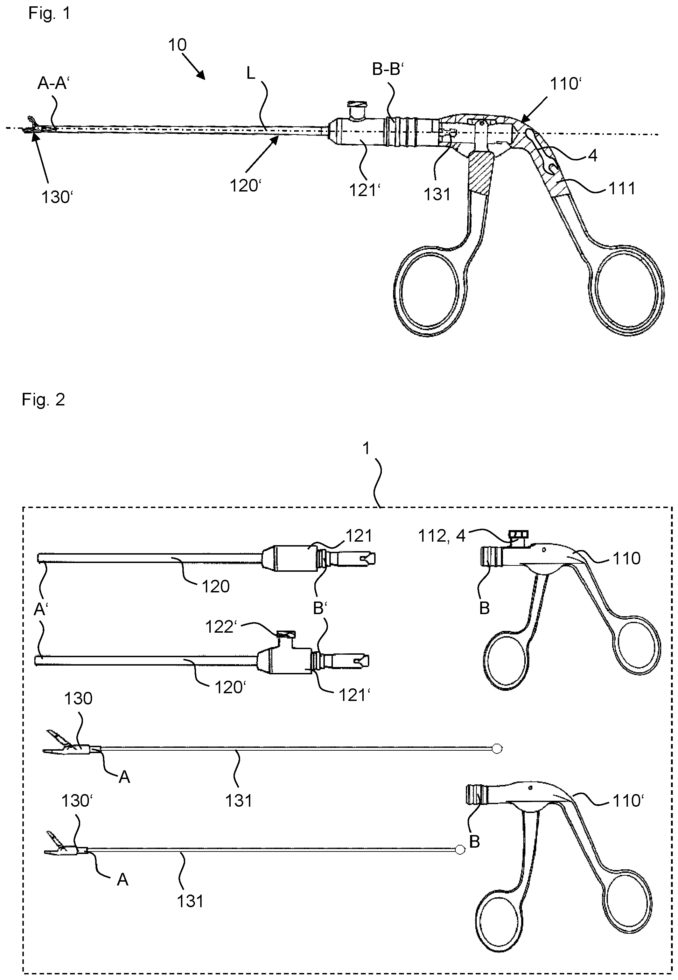

**Fig. 1** zeigt beispielhaft ein modulares chirurgisches Instrument 10, das zusammengesetzt ist aus den drei Komponenten eines chirurgischen Instrumentensystems 1, für das in **Fig. 2** ein einfaches Beispiel gezeigt ist. Das vereinfacht dargestellte modulare chirurgische Instrumentensystem 1 weist zwei unterschiedliche Griffe 110, 110', zwei Schäfte 120, 120' und zwei Werkzeugspitzen 130, 130' auf, die hier mit jeweils einem stangenförmigen Übertragungselement 131 verbunden sind. Die Schäfte 120, 120' des chirurgischen Instrumentensystems 1 weisen proximale Endstücke 121, 121' auf, wobei das proximale Endstück 121' des Schafts 120' einen Spülanschluss 122' aufweist. Der Handgriff 110 weist in diesem Beispiel eine Rändelschraube mit Drehknopf 112 als Funktionselement zum Sichern eines Schafts 120, 120' bzw. dessen Endstücks 121, 121' im Handgriff 110 auf. Die Werkzeugspitzen 130, 130' und die distalen Enden der Schäfte 120, 120' sind als distale Verbindungspartner A, A' für einen lösbaren Eingriff A-A' ausgebildet, und proximalen Enden der Schäfte 120, 120' am proximalen Endstück 121, 121' sind mit den Handgriffen 110,110' als proximale Verbindungspartner B, B' für einen lösbaren Eingriff B-B' ausgebildet.

Das aus dem Instrumentensystem 1 zusammenbaubare beispielhafte chirurgische Instrument 10 in **Fig. 1** weist die Instrumentenkomponenten Handgriff 110', Schaft 120' mit proximalem Endstück 121' und Werkzeugspitze 130' auf. Die Werkzeugspitze 130° als distaler Verbindungspartner A ist an einem distalen Ende A' des Schafts 120' angeordnet, der eine Längsachse L des chirurgischen Instruments 10 definiert. Die Werkzeugspitze 130' kann über das längsaxial im Schaft 120' bewegbare Übertragungselement 131 betätigt werden, das mit dem Handgriff 110', der als proximaler Verbindungspartner B an einem proximalen Ende B' des Schafts 120' angeordnet ist, in Wirkverbindung gebracht wird. Selbstverständlich kann aus dem chirurgischen Instrumentensystem 1 auch ein chirurgisches Instrument 10 mit den jeweils anderen Instrumentenkomponenten Handgriff 110, Schaft 120 und Werkzeugspitze 130 zusammengesetzt werden, sowie chirurgische Instrumente 10 mit jeder beliebigen Kombination der Instrumentenkomponenten Handgriffe 110, 110', Schäfte 120, 120' und Werkzeugspitzen 130, 130'. Ferner kann ein erfindungsgemäßes Instrumentensystem 1 selbstverständlich auch, anders als in **Fig. 2** dargestellt, mehr als zwei Sets der Instrumentenkomponenten, d. h. mehr als jeweils zwei Werkzeugspitzen, Schäfte und Handgriffe aufweisen, die sich beliebig zu mehr als zwei chirurgischen Instrumenten 10 zusammensetzen lassen. Ein Set besteht folglich aus einer Werkzeugspitze, einem Schaft und einem Handgriff, die zu einem chirurgischen Instrument 10 zusammengesetzt werden können.

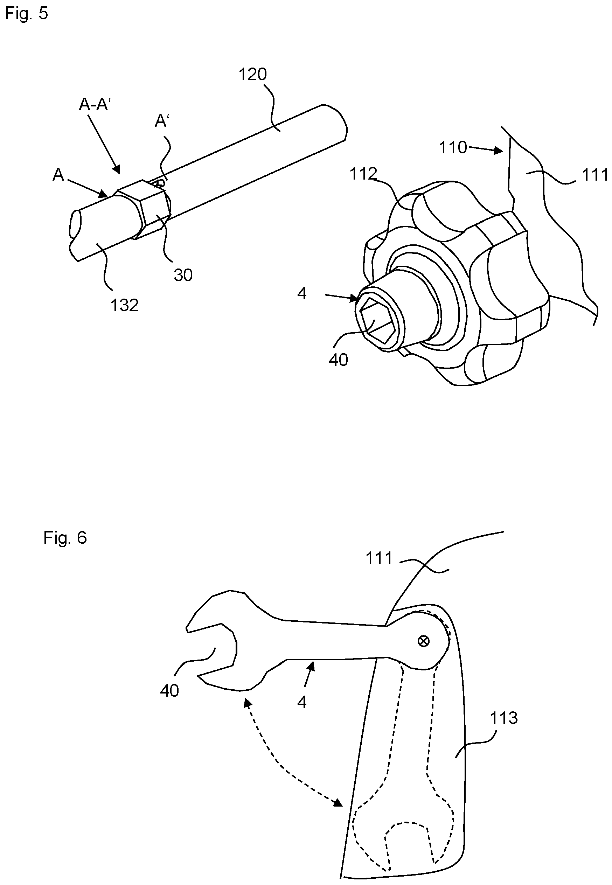

Zumindest eine der Komponenten des chirurgischen Systems 1 - in den Beispielen von **Fig. 1** und **Fig. 2** ist dies Handgriff 110, 110' - weist ein integriertes Montagewerkzeug 4 auf. In **Fig. 1** ist zu sehen, dass es sich bei dem Montagewerkzeug 4 des Handgriffs 110' um ein schwenkbar im Griffgehäuse 111 gelagertes Werkzeugelement handelt, das bei Nichtgebrauch in einem Aufnahmefach 113 im Griffgehäuse 111 Aufnahme findet (vgl. **Fig. 6****)** und das in diesem Beispiel als Maulschlüssel mit einem Schlüsselmaul als Eingriffsprofil 40 ausgebildet ist. Das Montagewerkzeug 4 des weiteren Handgriffs 110 des Instrumentensystems 1 in **Fig. 2** wird durch eine in den Drehknopf der Rändelschraube 112 integrierte Werkzeugfunktionalität bereitgestellt. Das Funktionselement Rändelschraube 112 hat als Basisfunktion die Sicherung des proximalen Schaftendes bzw. des proximalen Endstücks im Handgriff 110. Die davon abweichende, in den Drehknopf 112 integrierte Werkzeugfunktionalität kann wie im Beispiel von **Fig. 5** in einem Innensechskant als Eingriffsprofil 40 bestehen, das in dem Drehknopf 112 ausgeformt ist.

Dabei kann in einer Ausführungsform des Instrumentensystems 1 vorgesehen sein, dass jeweils eine Komponente jedes Sets bzw. alle gleichartigen Instrumentenkomponenten, z. B. alle Griffe, jeweils ein integriertes Montagewerkzeug 4 aufweisen, sodass jedes aus dem Instrumentensystem 1 zusammenbaubare chirurgische Instrument 10 erfindungsgemäß ein integriertes Montagewerkzeug 4 aufweist.

Durch den Einsatz eines Montagewerkzeugs zur Zerlegung der Verbindung zwischen den Komponenten Werkzeugspitze-Schaft und/oder Schaft-Handgriff kann die mechanische Stabilität der jeweiligen Verbindung bei gleichzeitig einfacherer Zerlegung erhöht werden und je nach Ausführungsform das Spiel der Komponenten reduziert werden. Grundsätzlich kann das Montagewerkzeug in eine der drei Komponenten des chirurgischen Instrumentensystems integriert werden, um die Verbindung der anderen beiden Komponenten zu fügen und wieder zu lösen. Dazu ist das Montagewerkzeug zur Zusammenwirkung mit einem der distalen Verbindungspartner und/oder einem der proximalen Verbindungspartner ausgebildet, um den Eingriff des zusammenwirkenden distalen Verbindungspartners und/oder den Eingriff des zusammenwirkenden proximalen Verbindungspartners zu lösen oder zu fügen.

In **Figuren 1-5** bezeichnet A-A' einen manuell oder mit dem Montagewerkzeug 4 lösbaren Eingriff der Werkzeugspitze 130, 130' als distalen Verbindungspartner A mit dem distalen Ende des Schafts 120, 120' als korrespondierenden distalen Verbindungspartner A'. Entsprechend bezeichnet B-B' einen manuell oder mit dem Montagewerkzeug 4 lösbaren Eingriff des Handgriffs 110, 110' als proximalen Verbindungspartner B mit dem proximalen Ende des Schafts 120, 120' als korrespondierenden proximalen Verbindungspartner B'.

Bei einem dreiteiligen Instrument 10 liegt das integrierte Montagewerkzeug 4 sinnvoller Weise zumindest an einer der endständigen Instrumentenkomponenten vor, d. h. an der Werkzeugspitze 130, 130°, die ein distaler Verbindungspartner A zum Eingriff A-A' mit dem distalen Schaftende A' ist, und/oder vorzugsweise an dem proximalen Handgriff 110, 110', der ein proximaler Verbindungspartner B zum Eingriff B-B' mit dem proximalen Schaftende B' ist. Das Montagewerkzeug 4 ist dabei zum Lösen und Fügen des Eingriffs der anderen endständigen Instrumentenkomponente, die das Montagewerkzeug 4 nicht aufweist, mit dem Schaft 120, 120' vorgesehen. Dazu ist das Montagewerkzeug 4 zum Zusammenwirken mit der anderen endständigen Instrumentenkomponente oder dem zugeordneten Schaftende bzw. einem zusätzlichen der Instrumentenkomponente oder dem Schaftende zugeordneten Verbindungselement wie beispielsweise einer Überwurfmutter ausgebildet.

Der Eingriff der jeweiligen Instrumentenkomponente, Werkzeugspitze 130, 130' oder Handgriff 110, 110', die das integrierte Montagewerkzeug 4 aufweist, mit dem Schaft 120, 120' kann in einer bevorzugten Ausführungsform manuell ohne Hilfsmittel lösbar ausgebildet sein, sodass jedes individuell zusammengebaute chirurgische Instrument 10 ohne weitere Hilfsmittel zerlegt und gefügt werden kann.

Alternativ könnte der Eingriff der Instrumentenkomponente, die das integrierte Montagewerkzeug 4 aufweist, mit dem Schaft 120, 120' ebenfalls als werkzeuglösbarer Eingriff ausgeführt sein, der der Verbindung eine erhöhte mechanische Stabilität und weniger Spiel verleiht. Da ein Eingriff zwischen Verbindungspartnern, von denen einer die Instrumentenkomponente mit dem Montagewerkzeug 4 ist, nicht durch das fest mit der Komponente verbundene Montagewerkzeug 4 gelöst werden kann, kann in einer weiteren Ausführungsform vorgesehen sein, dass das Montagewerkzeug 4 lösbar in die Instrumentenkomponente integriert ist. Ferner könnte die andere endständige Instrumentenkomponente oder eine weitere Instrumentenkomponente des chirurgischen Instrumentensystems 1, die nicht beim Zusammenbau des chirurgischen Instruments verwendet wird, ein (zusätzliches) Montagewerkzeug aufweisen, um den werkzeuglösbaren Eingriff der Instrumentenkomponente, die das Montagewerkzeug 4 aufweist, zu lösen.

In einer weiteren Ausführung, in der ein modulares Instrumentensystem 1 zumindest zwei Sets mit jeweils drei Instrumentenkomponenten aufweist, ist es denkbar, dass mit einem Montagewerkzeug 4 an einer der Komponenten alle Eingriffe zwischen den Verbindungspartnern jedes chirurgischen Instruments des chirurgischen Systems 1 gelöst (sowie gefügt) werden können, indem mit dem Montagewerkzeug 4 an der Komponente des ersten Sets die Eingriffe der Komponenten am zweiten Set gelöst werden und umgekehrt.

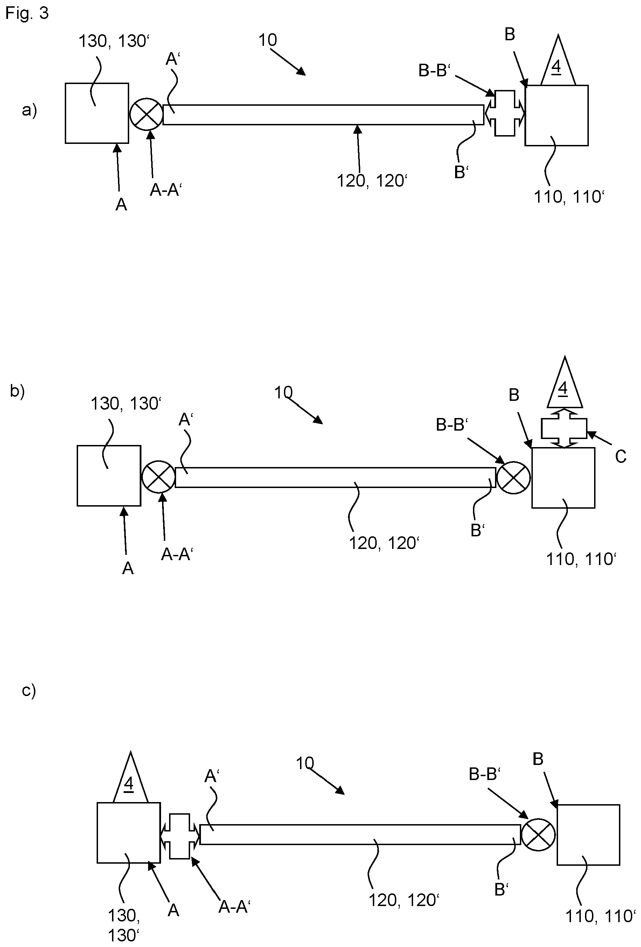

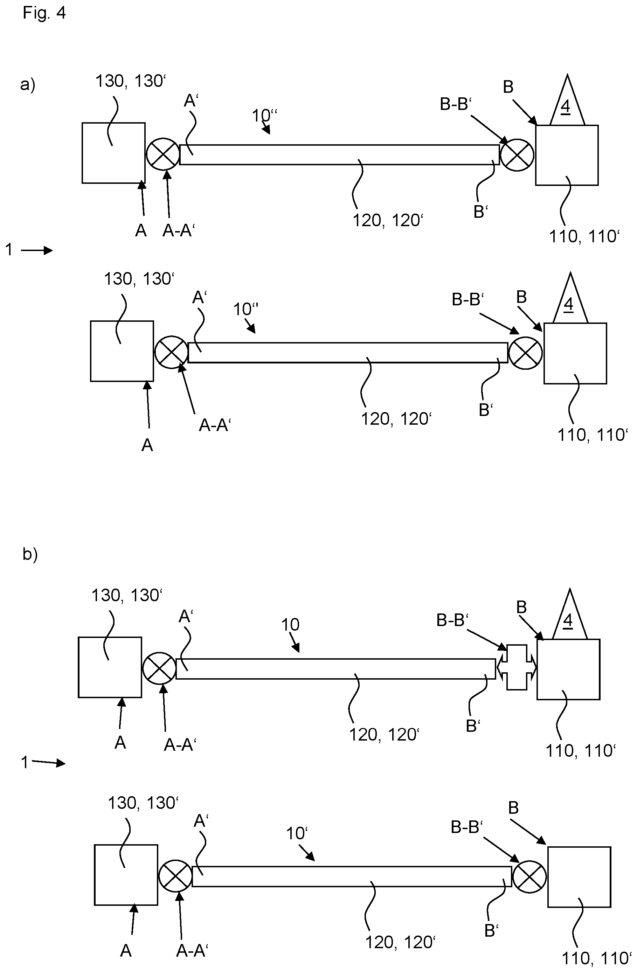

In **Figuren 1****,** **3** **und** **4** weist das erfindungsgemäße chirurgische Instrument 10 als aus einem Instrumentensystem 1 ausgewählten Komponenten jeweils eine Werkzeugspitze 130, 130', einen Schaft 120, 120' und einen Handgriff 110, 110' auf, wobei ein proximales Endstück 121' des Schafts 120' in **Fig. 1** dargestellt ist; in den schematischen **Fig. 3** **und** **4** wurde auf die Darstellung eines proximalen Endstücks verzichtet, kann aber als umfasst angesehen werden. Unter dem Ausdruck "Verbindung des Handgriffs mit dem Schaft" ist folglich nicht nur eine Verbindung des Handgriffs mit einem proximalen Endes des Schafts, sondern auch eine Verbindung des Handgriffs mit dem proximalen Endstück des Schafts mitzulesen.

In **Fig. 3** sind drei Varianten a, b und c eines erfindungsgemäßen chirurgischen Instruments 10 gezeigt, das sich aus einem erfindungsgemäßen Instrumentensystem 1 zusammenbauen lässt: In **Fig. 3a** ist das Montagewerkzeug 4 in den Handgriff 110, 110' integriert, um den Eingriff A-A' der distalen Verbindungspartner A, A', d. h. der Werkzeugspitze 130, 130' mit dem distalen Ende des Schafts 120, 120', zu lösen (bzw. auch wieder zu fügen). Der Eingriff B-B' der proximalen Verbindungspartner B, B', d. h. des Handgriffs 110, 110' mit dem Schaft 120, 120° (bzw. dem nicht dargestellten proximalen Endstück), ist hierbei händisch lösbar (z. B. durch Betätigung einer Rändelschraube mit Drehknopf 112), sodass nach Lösen des Handgriffs 110, 110' vom Schaft 120, 120' das Montagewerkzeug 4 am Handgriff 110, 110' zur Verfügung steht, um den distalen Eingriff A-A' der Werkzeugspitze 130, 130' mit dem Schaft 120, 120' zu lösen. Diese Variante stellt eine bevorzugte Ausführungsform dar, da ein Handgriff 110, 110' breite Möglichkeiten zur Integration eines Montagewerkzeugs 4 bietet und die manuelle Lösbarkeit des Eingriffs B-B' keine weiteren Montagewerkzeuge erforderlich macht.

In einem Fall, bei dem sowohl der Eingriff A-A' der Werkzeugspitze 130, 130' mit dem Schaft 120, 120° als auch der Eingriff B-B' des Schafts 120, 120' mit dem Handgriff 110, 110' als werkzeuglösbare Verbindungen ausgeführt sind, kann das Montagewerkzeuge 4 händisch lösbar in die jeweilige Instrumentenkomponente integriert sein. Eine solche Variante ist im Beispiel des chirurgischen Instruments 10 in **Fig. 3b** mit der manuell lösbaren Anordnung C des Montagewerkzeugs 4 am Handgriff 110, 110' dargestellt. Für eine händisch lösbare Anordnung C des Montagewerkzeugs 4 kann der Handgriff 110, 110' beispielsweise ein Aufnahmefach aufweisen, das korrespondierend zu dem Montagewerkzeug 4 als Steckeinschub ausgeformt sein kann. Gegebenenfalls kann ferner in eine Instrumentenkomponente mehr als ein Montagewerkzeug 4 integriert sein, etwa wenn beide Eingriffe A-A' und B-B' werkzeuglösbar sind und unterschiedliche Montagewerkzeuge erfordern, wobei in einem solchen Fall zumindest eines der Montagewerkzeuge lösbar in der Instrumentenkomponente integriert ist.

**Fig. 3c** zeigt eine weniger bevorzugte Ausführungsform, bei der ein zum Lösen und Fügen des proximalen Eingriffs B-B' des Schafts 120, 120' mit dem Handgriff 110, 110' vorgesehenes Montagewerkzeug 4 in die Werkzeugspitze 130, 130' integriert ist. Funktion und Abmessungen der Werkzeugspitze 130, 130' beschränken allerdings die Möglichkeiten zur Integration eines Montagewerkzeugs 4. Der distale Eingriff A-A' der Werkzeugspitze 130, 130' mit dem Schaft 120, 120' ist hierbei händisch zu fügen und zu lösen (z. B. durch einen Bajonett- und/oder Rasteingriff), sodass nach Lösen der Werkzeugspitze 130, 130' vom Schaft 120, 120' das Montagewerkzeug 4 an der Werkzeugspitze 130, 130' zur Verfügung steht, um den Eingriff B-B' des Handgriffs 110, 110' mit dem Schaft 120, 120' zu lösen bzw. zu fügen.

Das Zerlegekonzept eines erfindungsgemäßen modularen chirurgischen Instrumentensystems 1 mit zumindest zwei Instrumentensets aus Werkzeugspitze, Schaft und Handgriff kann vorsehen, dass jeweils eine Instrumentenkomponente jedes Sets ein Montagewerkzeug 4 in einer der vorbeschriebenen Ausführungsformen aufweisen kann, sodass jedes aus dem modularen chirurgischen Instrumentensystem 1 zusammengebaute chirurgische Instrument 10 eine Instrumentenkomponente mit integriertem Montagewerkzeug 4 zur Demontage entsprechend einer der in **Fig. 3a, b, c** gezeigte Varianten aufweist.

**Fig. 4** veranschaulicht in zwei Beispielen a) und b) weitere Zerlegekonzepte für chirurgische Instrumente 10, 10', 10" eines modularen Instrumentensystems 1, das zumindest zwei Instrumentensets mit Werkzeugspitzen 130, 130', Schäften 120, 120' und Handgriffen 110, 110' umfasst. **Fig. 4a** verdeutlicht eine weitere Ausführungsform eines erfindungsgemäßen Instrumentensystems 1, bei dem sich aus den Instrumentenkomponenten zwei werkzeuglösbare chirurgische Instrumente 10" zusammenbauen lassen, bei denen jeweils beide Eingriffe A-A' und B-B' als werkzeuglösbare Verbindungen gestaltet sind. In der in **Fig. 4b** gezeigten weiteren alternativen Ausführungsform eines erfindungsgemäßen Instrumentensystems 1 lassen sich aus den Instrumentenkomponenten des Instrumentensystems 1 ein erstes chirurgisches Instrument 10, das eine Instrumentenkomponente mit einem Montagewerkzeug 4 aufweist, und ein zweites werkzeugloses chirurgisches Instrument 10' zusammenbauen, bei dem keine der Instrumentenkomponenten ein Montagewerkzeug aufweist.

Im Beispiel von **Fig. 4a****,** das zwei werkzeuglösbare chirurgische Instrumente 10" eines Instrumentensystems 1 zeigt, sind sowohl der distale Eingriff A-A' von Schaft 120, 120' und Werkzeugspitze 130, 130' als auch der proximale Eingriff B-B' von Schaft 120, 120' und Handgriff 110, 110' als werkzeuglösbare Verbindung ausgebildet. Ferner weist jeder Handgriff 110, 110' jeweils ein nicht-lösbar vorliegendes Montagewerkzeug 4 auf, das sowohl zum Lösen des Eingriffs A-A' von Schaft 120, 120' und Werkzeugspitze 130, 130' als auch zum Lösen des Eingriffs B-B' von Schaft 120, 120' und Handgriff 110, 110' ausgebildet ist. Damit kann das Montagewerkzeug 4 jedes Handgriffs 110, 110' (sowohl im unverbauten als auch in einem zu einem werkzeuglösbaren Instrument 10" verbauten Zustand) verwendet werden, um die die Eingriffe A-A'. B-B' des jeweils anderen werkzeuglösbaren chirurgischen Instruments 10" zu lösen (bzw. zu fügen). In einer nicht dargestellten, dazu alternativen Ausführungsform können die Handgriffe 110, 110' des Instrumentensystems 1 jeweils zwei Montagewerkzeuge 4 aufweisen, von denen eines zum Lösen und Fügen des distalen Eingriffs A-A' und ein zweites zum Lösen des proximalen Eingriffs B-B' vorgesehen sein kann.

Bei den in **Fig. 4b** gezeigten chirurgischen Instrumenten 10, 10' weist das erste chirurgische Instrument 10 entsprechend **Fig. 3a** einen händisch lösbaren Eingriff B-B' eines Schaft 120, 120' mit einem Handgriff 110, 110' auf, der das integrierte Montagewerkzeug 4 aufweist. Mit diesem Montagewerkzeug 4 lässt sich nun nicht nur der distale Eingriff A-A' des Schaft 120, 120' mit der Werkzeugspitze 130, 130' des ersten chirurgischen Instruments 10 lösen, sondern auch der distale Eingriff A-A' und der proximale Eingriff B-B' des zweiten, werkzeuglosen chirurgischen Instruments 10'. Nicht dargestellt sind dazu mögliche Alternativen, bei denen das erste chirurgische Instrument 10 entsprechend einem der Beispiele in **Fig. 3b oder 3c** ausgeführt sein kann.

Bei einem Zerlegekonzept entsprechend **Fig. 4a** mit zumindest zwei Instrumentensets können selbstverständlich auch Varianten umfasst sein, bei denen ein integriertes Montagewerkzeug am Schaft 120, 120' bzw. insbesondere an dessen proximalen Endstück 121, 121', oder ggf. an der Werkzeugspitze 130, 130' vorgesehen sein kann, um die Eingriffe A-A', B-B' eines weiteren, aus dem Instrumentensystem 1 zusammengebauten Instruments 10" zu lösen. Eine Integration des Montagewerkzeugs 4 in den Schaft 120, 120' bzw. dessen proximales Endstück 121, 121' ist hierbei möglich, da mit dem Montagewerkzeug 4 des einen chirurgischen Instruments 10" die Eingriffe A-A', B-B' des anderen chirurgischen Instruments 10" gelöst werden.

Selbstverständlich kann ein modulares Instrumentensystem auch mehr als zwei Sets, d. h. mehr als jeweils zwei Werkzeugspitzen, Schäfte und Handgriffe aufweisen, die sich beliebig zu mehr als zwei chirurgischen Instrumenten zusammensetzen lassen. Ferner muss die Anzahl der jeweiligen Komponenten Werkzeugspitzen, Schäften und Handgriffen nicht gleich sein; beispielsweise könnte die Anzahl der Handgriffe oder Werkzeugspitzen die Anzahl der Schäfte übersteigen.

Beispiele für integrierte Montagewerkzeuge sind Schraubenschlüssel wie Maulschlüssel, Ringschlüssel, Ratschenringschlüssel, Steckschlüsseleinsätze mit Außen- oder Innensechskant, Außen- oder Innensechsrund (TORX^{®}), Außen- oder Innenvielzahn sowie Schlitz-, Kreuzschlitzschraubenzieher und Zangen. Weitere, auch individuell angepasste Montagewerkzeuge sind ebenfalls denkbar. Jedes Montagewerkzeug kann in Form eines Eingriffsprofils, das in ein Funktionselement der Komponente (z. B. Griffgehäuse, Drehknopf etc.) integral eingebracht ist, oder als Werkzeugelement vorliegen, das wie bei einem Schweizer Taschenmesser in einem Aufnahmefach schwenkbar angelenkt oder heraus- und einschiebbar in der jeweiligen Instrumentenkomponente (z. B. Handgriff) aufgenommen ist.

**Fig. 5** zeigt beispielhaft ein als in den Handgriff 110 (dessen Griffgehäuse 111 nur angedeutet ist) integriertes Montagewerkzeug 4 mit einem Innensechskant als Eingriffsprofil 40, das in dem Drehknopf 112 einer Rändelschraube ausgeformt ist. Die Rändelschraube kann zum händischen Sichern des proximalen Endes des Schafts 120 im Handgriff 110 vorgesehen sein. Mit dem Innensechskant-Montagewerkzeug 4 am Handgriff 110 kann der Eingriff A-A' gelöst werden, der in diesem Beispiel als Schraubverbindung zwischen der Werkzeugspitze 130, von der in **Fig. 5** nur der distale Ansatz eines Werkzeug- bzw. Zangeneinsatz 132 dargestellt ist, und dem distalen Ende des Schafts 120 ausgeführt ist. Dazu weist ein dem Werkzeug- bzw. Zangeneinsatz 132 zugeordnetes zusätzliches Verbindungselement ein entsprechendes Mitnahmeprofil 30 auf, das im dargestellten Beispiel eine Sechskantmutter bzw. Überwurfmutter ist.

Ein aus einem Aufnahmefach 113 im Griffgehäuse 111 ausgeschwenktes Montagewerkzeug 4 ist in **Fig. 6** dargestellt. Ferner ist gestrichelt das Montagewerkzeug 4 eingeschwenkt in das Aufnahmefach 113 angedeutet. Das Montagewerkzeug 4 ist hier ein Maulschlüssel mit einem Schlüsselmaul als Eingriffsprofil 40, das sich ebenfalls zum Lösen einer Schraubverbindung mit einem entsprechenden Mitnahmeprofil eignet.

Selbstverständlich sind von den gezeigten Beispielen abweichende, hier nicht figurativ dargestellte Modifikationen des Montagewerkzeugs mit anderen der oben angeführten Eingriffsprofile zum Lösen, Fügen entsprechender Mitnahmeprofile ebenfalls umfasst.

Nicht dargestellt, aber ebenfalls vom Schutzumfang umfasst, sind weitere Ausführungsformen, die beispielsweise beide vorgenannten Montagewerkzeugvarianten aufweisen können, oder die ein von der Komponente lösbares Montagewerkzeug umfassen, das beispielsweise in einem entsprechend geformten Einschubfach eingesteckt und herausgezogen werden kann.

Die Schritte eines erfindungsgemäßen Verfahrens zur Montage und Demontage beziehen sich auf den Zusammenbau der Instrumentenkomponenten eines erfindungsgemäßen Instrumentensystems 1, um ein anwendungsfertiges chirurgisches Instrument 10, 10', 10" bereitzustellen, sowie auf das Zerlegen des jeweiligen chirurgischen Instruments 10, 10', 10" in die Instrumentenkomponenten, um diese nach Gebrauch zur Wiederverwendung reinigen und desinfizieren zu können. Sowohl beim Zusammenbau als auch beim Zerlegen wird die Instrumentenkomponente mit dem Montagewerkzeug 4 bereitgestellt, das bei der Montage des chirurgischen Instruments 10, 10', 10" dazu verwendet wird, zumindest einen Eingriff - den Eingriff A-A' der distalen Verbindungspartner A, A' oder den Eingriff B-B' der proximalen Verbindungspartner B, B' - herzustellen. Derselbe Eingriff A-A' oder B-B' wird bei der Demontage des chirurgischen Instruments 10, 10', 10" durch Verwendung des Montagewerkzeugs 4 entsprechend gelöst. Welcher Eingriff mit dem Montagewerkzeug gelöst und gefügt werden kann, und auch weitere Schritte des Verfahrens hängen von der Ausführungsform des erfindungsgemäßen modularen chirurgischen Instrumentensystems 1 und der damit zusammenbaubaren Instrumente 10, 10', 10" ab.

Weist ein erfindungsgemäßes Instrumentensystem 1 Instrumentenkomponenten zum Zusammenbau eines chirurgischen Instruments 10 gemäß Fig. 3a auf, umfasst das Verfahren zur Montage die Schritte:
- Bereitstellen des Handgriffs 110, 110', der das integrierte Montagewerkzeug 4 aufweist, das zum Zusammenwirken mit zumindest einem der distalen Verbindungspartner A, A' (Werkzeugspitze 130, 130' und distales Ende des Schafts 120, 120°) ausgebildet ist; und
- unter Verwendung des Montagewerkzeugs 4 Herstellen des Eingriffs A-A' der distalen Verbindungspartner A, A', sodass die Werkzeugspitze 130, 130° mit dem distalen Ende des Schafts 120, 120' mechanisch stabil mit reduziertem Spiel verbunden ist;
- Beenden der Montage und Erhalten des anwendungsfertigen chirurgischen Instruments 10 durch manuell Herstellen des Eingriffs B-B' der proximalen Verbindungspartner B, B', wobei das proximale Ende des Schafts 120, 120' manuell ohne Werkzeug mit dem Handgriff 110, 110' verbunden wird, der das integrierte Montagewerkzeug 4 aufweist.

Zur Demontage dieses chirurgischen Instrument 10 weist das Verfahren die Schritte auf:
- Bereitstellen des Handgriffs 110, 110', der das integrierte Montagewerkzeug 4 aufweist, durch manuell ohne Werkzeug Lösen des Eingriffs B-B' der proximalen Verbindungspartner B, B', wobei der Handgriff 110, 110', der das integrierte Montagewerkzeug 4 aufweist, von dem proximalen Ende des Schafts 120, 120` getrennt wird,
- unter Verwendung des Montagewerkzeugs 4 Lösen des Eingriffs A-A' der distalen Verbindungspartner A, A', sodass die Werkzeugspitze 130, 130' von dem distalen Ende des Schafts 120, 120' getrennt wird;
- Beenden der Demontage und Erhalten getrennter Instrumentenkomponenten zur Weiterbehandlung, die für die Wiederverwendung ein Reinigen und Desinfizieren umfassen kann.

Die Verfahrensschritte zur Montage und Demontage eines chirurgischen Instruments 10 gemäß **Fig. 3c** weichen davon dahingehend ab, dass die Instrumentenkomponente, die das Montagewerkzeug 4 aufweist, die Werkzeugspitze 130, 130' ist, deren Eingriff A-A' mit dem distalen Ende des Schafts 120, 120' händisch lösbar ausgebildet ist, während das Montagewerkzeug 4 zum Zusammenwirken mit einem der proximalen Verbindungspartner B, B', also dem proximalen Endes des Schafts 120, 120' und dem Handgriff 110, 110' ausgebildet ist. Bei der Montage wird die Werkzeugspitze 130, 130' mit dem Montagewerkzeug 4 bereitgestellt, um den Eingriff B-B' zwischen dem proximalen Ende des Schafts 120, 120' und dem Handgriff 110, 110' zu fügen, wonach die Werkzeugspitze 130, 130' manuell in Eingriff A-A' mit dem distalen Ende des Schafts 120, 120' gebracht wird, sodass das anwendungsfertige chirurgische Instrument 10 bereitgestellt ist. Bei der Demontage wird der Eingriff A-A' der Werkzeugspitze 130, 130' mit dem distalen Ende des Schafts 120, 120' gelöst, um die Instrumentenkomponente mit dem Montagewerkzeug 4 bereitzustellen, mit dem der Eingriff B-B' zwischen dem proximalen Ende des Schafts 120, 120' und dem Handgriff 110, 110' gelöst wird, um die getrennten Instrumentenkomponenten für eine Wiederverwendung weiterbehandeln zu können.
- Leicht abweichend dazu weist das Verfahren zur Montage eines chirurgischen Instruments 10 gemäß **Fig. 3b****,** das in entsprechender Weise auch für Ausführungsformen gilt, bei denen das lösbare Montagewerkzeug 4 nicht am Handgriff, sondern an der Werkzeugspitze oder dem Schaft oder dessen proximalen Endstück angeordnet ist, die Schritte auf:
- Bereitstellen des Handgriff 110, 110' mit einem lösbaren Montagewerkzeug 4, das zum Zusammenwirken mit einem der distalen Verbindungspartner A, A' und zum Zusammenwirken mit einem der Verbindungspartner B, B' ausgebildet ist;
- manuell Lösen des Montagewerkzeugs 4 von dem Handgriff 110, 110';
- unter Verwendung des Montagewerkzeugs 4 Herstellen des Eingriffs A-A' der distalen Verbindungspartner A, A' und Herstellen des Eingriffs B-B' der proximalen Verbindungspartner B, B', sodass die Werkzeugspitze 130, 130° mit dem distalen Ende des Schafts 120, 120' und das proximale Ende des Schafts 120, 120° mit dem Handgriff 110, 110' mechanisch stabil mit reduziertem Spiel verbunden sind;
- Beenden der Montage und Erhalten des anwendungsfertigen chirurgischen Instruments 10 durch manuell Anordnen des Montagewerkzeugs 4 am Handgriff 110, 110'.

Zur Demontage dieses chirurgischen Instrument 10 weist das Verfahren die Schritte auf:
- manuell Lösen des Montagewerkzeugs 4 von dem Handgriff 110, 110';
- unter Verwendung des Montagewerkzeugs 4 Lösen des Eingriffs A-A' der distalen Verbindungspartner A, A' und des Eingriffs B-B' der distalen Verbindungspartner B, B' sodass die Werkzeugspitze 130, 130' von dem distalen Ende des Schafts 120, 120' und das proximale Ende des Schafts 120, 120' von dem Handgriff 110, 110' getrennt werden;
- Bereitstellen des Handgriffs 110, 110', der das integrierte Montagewerkzeug 4 aufweist, durch manuell Anordnen des Montagewerkzeugs 4 am Handgriff 110, 110';
- Beenden der Demontage und Erhalten getrennter Instrumentenkomponente zur Weiterbehandlung, die für die Wiederverwendung ein Reinigen und Desinfizieren umfassen kann.

Weitere Ausführungsformen des erfindungsgemäßen Verfahrens beziehen sich auf die erfindungsgemäßen Instrumentensysteme gemäß **Fig. 4a und 4b****.** Das chirurgische Instrumentensystem 1 gemäß **Fig. 4b** enthält zumindest ein Set aus Instrumentenkomponenten für ein erstes chirurgisches Instrument 10 gemäß den Beispielen aus **Fig. 3a, b, c,** sodass in Bezug auf die Verfahrensschritte zur Montage und Demontage dieses erste chirurgische Instrument 10 obige Ausführungen entsprechend gelten. Darüber hinaus weist das chirurgische Instrumentensystem 1 gemäß **Fig. 4b** zumindest ein Set aus Instrumentenkomponenten für ein zweites chirurgisches Instrument 10' auf, bei dem keine Instrumentenkomponente ein Montagewerkzeug aufweist. Dabei sind die Eingriffe A-A' und B-B' zwischen den Instrumentenkomponenten dieses zweiten, werkzeuglosen chirurgischen Instruments 10° zum Zusammenwirken mit dem Montagewerkzeug 4 des ersten chirurgischen Instruments 10 ausgebildet, sodass ein Verfahren zur Montage des zweiten werkzeuglosen chirurgischen Instruments 10' die Schritte umfasst:
- Bereitstellen des Handgriffs 110, 110' des ersten chirurgischen Instruments 10, der das integrierte Montagewerkzeug 4 aufweist; und
- unter Verwendung des Montagewerkzeugs 4 des ersten chirurgischen Instruments 10 Herstellen des Eingriffs A-A' der distalen Verbindungspartner A, A' des zweiten, werkzeuglosen chirurgischen Instruments 10' und Herstellen des Eingriffs B-B' der proximalen Verbindungspartner B-B' des zweiten, werkzeuglosen chirurgischen Instruments 10'; wobei das anwendungsfertige zweite, chirurgische Instrument 10' erhalten wird.

Entsprechend weist das Verfahren zur Demontage eines solchen werkzeuglosen chirurgischen Instruments 10' die Schritte auf:
- Bereitstellen des Handgriffs 110, 110' des ersten chirurgischen Instruments 10, der das integrierte Montagewerkzeug 4 aufweist,
- unter Verwendung des Montagewerkzeugs 4 Lösen des Eingriffs A-A' der distalen Verbindungspartner A, A' des zweiten, werkzeuglosen chirurgischen Instruments 10' und Lösen des Eingriffs B-B' der proximalen Verbindungspartner B-B' des zweiten, werkzeuglosen chirurgischen Instruments 10';
wobei die getrennten Instrumentenkomponenten zur Weiterbehandlung erhalten werden.

Zum Zusammenbau zumindest zweier chirurgischer Instrumente 10" gemäß **Fig. 4a** weist ein erfindungsgemäßes Instrumentensystem 1 zumindest einen ersten und zweiten Handgriff 110, 110°, einen ersten und zweiten Schaft 120, 120' und eine erste und zweite Werkzeugspitze 130, 130° als Instrumentenkomponenten auf. Bei diesen chirurgischen Instrumenten 10" sind alle Eingriffe A-A', B-B' als werkzeuglösbare (bzw. fügbare) Verbindungen ausgebildet, wobei das Montagewerkzeug 4 nicht-lösbar an einer Instrumentenkomponente vorliegt. In diesem Beispiel weist jeder Handgriff 110, 110' ein nicht-lösbares Montagewerkzeug 4 auf. Das Verfahren zur Montage eines solchen werkzeuglösbaren chirurgischen Instruments 10" aus einem ersten Handgriff 110, 110', einem ersten Schaft 120, 120' und einer ersten Werkzeugspitze 130, 130' umfasst die Schritte:
- Bereitstellen des zweiten Handgriffs 110, 110', der das integrierte Montagewerkzeug 4 aufweist, in einem zu einem chirurgischen Instrument 10" verbauten oder unverbauten Zustand, wobei das Montagewerkzeug 4 des zweiten Handgriffs 110, 110' ausgebildet ist zum Zusammenwirken mit einem der distalen Verbindungspartner A, A', die die erste Werkzeugspitze 130, 130' und das distale Ende des ersten Schafts 120, 120' sind, und zum Zusammenwirken mit einem der proximalen Verbindungspartner B, B', die das proximale Ende des ersten Schafts 120, 120' und der erste Handgriff 110, 110' sind;
- unter Verwendung des Montagewerkzeugs 4 des zweiten Handgriffs 110, 110' Herstellen des Eingriffs A-A' der ersten Werkzeugspitze 130, 130' mit dem distalen Ende des ersten Schafts 120, 120';
- unter Verwendung des Montagewerkzeugs 4 des zweiten Handgriffs 110, 110' Herstellen des Eingriffs B-B' des proximalen Endes des ersten Schafts 120, 120' mit dem ersten Handgriff 110, 110'.

Die Schritte zur Montage eines weiteren werkzeuglösbaren chirurgischen Instruments 10" aus dem zweiten Handgriff 110, 110', dem zweiten Schaft 120, 120' und der zweiten Werkzeugspitze 130, 130' umfassen entsprechend die Verwendung des Montagewerkzeugs 4 des ersten Handgriffs 110, 110' zum Fügen der Eingriffe A-A', B-B' zwischen dem zweiten Handgriff 110, 110', dem zweiten Schaft 120, 120' und der zweiten Werkzeugspitze 130, 130'. Grundsätzlich erfolgt die Montage der werkzeuglösbaren chirurgischen Instrumente 10" immer durch Verwendung eines Montagewerkzeugs 4 einer Instrumentenkomponente, die nicht Teil des montierten werkzeuglösbaren chirurgischen Instruments 10" ist.

Entsprechend erfolgt auch die Demontage eines werkzeuglösbaren chirurgischen Instruments 10" immer durch ein Montagewerkzeug 4 einer Instrumentenkomponente, die nicht Teil des zu demontierenden werkzeuglösbaren chirurgischen Instruments 10" ist. In Bezug auf das Beispiel aus **Fig. 4a** umfassen die Schritte zur Demontage eines werkzeuglösbaren chirurgischen Instruments 10", das aus einem ersten Handgriff 110, 110', einem ersten Schaft 120, 120' und einer ersten Werkzeugspitze 130, 130' zusammengesetzt ist, entsprechend:
- Bereitstellen des zweiten Handgriffs 110, 110', der das integrierte Montagewerkzeug 4 aufweist, in einem zu einem chirurgischen Instrument 10" verbauten oder unverbauten Zustand, wobei das Montagewerkzeug 4 des zweiten Handgriffs 110, 110' ausgebildet ist zum Zusammenwirken mit einem der distalen Verbindungspartner A, A', die die erste Werkzeugspitze 130, 130' und das distale Ende des ersten Schafts 120, 120' sind, und zum Zusammenwirken mit einem der proximalen Verbindungspartner B, B', die das proximale Ende des ersten Schafts 120, 120' und der erste Handgriff 110, 110' sind;
- unter Verwendung des Montagewerkzeugs 4 des zweiten Handgriffs 110, 110' Lösen des Eingriffs A-A' der ersten Werkzeugspitze 130, 130' mit dem distalen Ende des ersten Schafts 120, 120';
- unter Verwendung des Montagewerkzeugs 4 des zweiten Handgriffs 110, 110' Lösen des Eingriffs B-B' des proximalen Endes des ersten Schafts 120, 120' mit dem ersten Handgriff 110, 110'.

Analog erfolgt die Demontage eines weiteren werkzeuglösbaren chirurgischen Instruments 10" aus dem zweiten Handgriff 110, 110', dem zweiten Schaft 120, 120' und der zweiten Werkzeugspitze 130, 130' unter Verwendung des Montagewerkzeugs 4 des ersten Handgriffs 110, 110' zum Lösen der Eingriffe A-A', B-B' zwischen dem zweiten Handgriff 110, 110', dem zweiten Schaft 120, 120' und der zweiten Werkzeugspitze 130, 130'.

Entsprechende Verfahrensschritte gelten für Abwandlungen dieser Ausführungsform des Instrumentensystems 1, bei denen jeder Handgriff zwei Montagewerkzeuge aufweist, jeweils eines für den jeweiligen Eingriff A-A', B-B', oder bei denen das Montagewerkzeug 4 nicht am Handgriff, sondern an der Werkzeugspitze oder dem Schaft bzw. dessen proximalen Endstück vorliegt.

### BEZUGSZEICHENLISTE

- 1: Modulares chirurgisches Instrumentensystem
- 10, 10', 10": Modulares chirurgisches Instrument
- 110, 110': Handgriff
- 111: Griffgehäuse
- 112: Drehknopf
- 113: Aufnahmefach, Aussparung
- 120, 120': Schaft
- 121, 121': Proximales Endstück
- 122': Spülanschluss
- 130, 130': Werkzeugspitze
- 131: Betätigungselement
- 132: Werkzeugeinsatz
- 30: Mitnahmeprofil (an einem der Verbindungspartner oder zusätzlichem Verbindungselement)
- 4: Montagewerkzeug
- 40: Eingriffsprofil

- A, A': Distale Verbindungspartner Werkzeugspitze und distales Ende des Schafts
- B, B': Proximale Verbindungspartner Handgriff und proximales Ende des Schafts
- C: Lösbare Anordnung des Montagewerkzeugs
- L: Längsachse

## Patentansprüche

1. Chirurgisches Instrumentensystem (1) mit den Instrumentenkomponenten:
- zumindest eine Werkzeugspitze (130, 130'),
- zumindest einen Schaft (120, 120') und
- zumindest einen Handgriff (110,110'), wobei
die Werkzeugspitze (130, 130') und ein distales Ende des Schafts (120, 120') als distale Verbindungspartner (A, A') ausgebildet sind, die in lösbarem Eingriff (A-A') verbindbar sind, und
ein proximales Ende des Schafts (120, 120') und der Handgriff (110,110') als proximale Verbindungspartner (B, B') ausgebildet sind, die in lösbarem Eingriff (B-B') verbindbar sind,
**dadurch gekennzeichnet, dass**
zumindest eine der Instrumentenkomponenten ein integriertes Montagewerkzeug (4) aufweist, das zur Zusammenwirkung mit zumindest einem der distalen Verbindungspartner (A, A') oder einem der proximalen Verbindungspartner (B, B') ausgebildet ist, um den Eingriff (A-A') des zusammenwirkenden distalen Verbindungspartners (A, A') und/oder den Eingriff (B-B') des zusammenwirkenden proximalen Verbindungspartners (B, B') zu lösen oder zu fügen.

2. Chirurgisches Instrumentensystem (1) nach Anspruch 1,
**dadurch gekennzeichnet, dass**
ein erstes chirurgisches Instrument (10), das aus zumindest einem ersten Set der Instrumentenkomponenten des chirurgischen Instrumentensystems (1) zusammenbaubar ist, die Instrumentenkomponente mit dem integrierten Montagewerkzeug (4) als einen der distalen Verbindungspartner (A, A') oder der proximalen Verbindungspartner (B, B') aufweist, wobei
- der Eingriff (A-A') oder der Eingriff (B-B') der Instrumentenkomponente, die das integrierte Montagewerkzeug (4) aufweist, mit dem jeweiligen distalen Verbindungspartner (A, A') oder proximalen Verbindungspartner (B, B'), der der Instrumentenkomponente mit dem integrierten Montagewerkzeug (4) zugeordnet ist, manuell lösbar und fügbar ist,
oder
- das Montagewerkzeug (4) manuell lösbar an der Instrumentenkomponente vorliegt und zur Zusammenwirkung mit einem der distalen Verbindungspartner (A, A') und einem der proximalen Verbindungspartner (B, B') ausgebildet ist, um den Eingriff (A-A') des zusammenwirkenden distalen Verbindungspartners (A, A') und den Eingriff (B-B') des zusammenwirkenden proximalen Verbindungspartners (B, B') zu lösen oder zu fügen.

3. Chirurgisches Instrumentensystem (1) nach Anspruch 2,
**dadurch gekennzeichnet, dass**
das chirurgische Instrumentensystem (1) zumindest ein zweites Set der Instrumentenkomponenten, aus denen ein zweites chirurgisches Instrument (10', 10") zusammenbaubar ist, und zumindest eine weitere Instrumentenkomponente aufweist, die eine Werkzeugspitze (130, 130') oder ein Schaft (120, 120') oder ein Handgriff (110,110') ist und die das integrierte Montagewerkzeug (4) aufweist, das zur Zusammenwirkung mit einem der distalen Verbindungspartner (A, A') und mit einem der proximalen Verbindungspartner (B, B') des zweiten chirurgischen Instruments (10', 10") ausgebildet ist, um den Eingriff (A-A') des zusammenwirkenden distalen Verbindungspartners (A, A') und den Eingriff (B-B') des zusammenwirkenden proximalen Verbindungspartners (B, B') des zweiten chirurgischen Instruments (10', 10") zu lösen oder zu fügen.

4. Chirurgisches Instrumentensystem (1) nach Anspruch 3,
**dadurch gekennzeichnet, dass**
das erste Set zum Zusammenbau des ersten chirurgischen Instruments (10) die weitere Instrumentenkomponente mit dem integrierten Montagewerkzeug (4) aufweist, und das zweite chirurgische Instrument (10') ein werkzeugloses chirurgisches Instrument (10') ist, das keine Instrumentenkomponente mit dem Montagewerkzeug (4) aufweist.

5. Chirurgisches Instrumentensystem (1) nach einem der Ansprüche 2 bis 4,
**dadurch gekennzeichnet, dass**
- die Instrumentenkomponente mit dem integrierten Montagewerkzeug (4) der Handgriff (110, 110') ist, dessen Eingriff (B-B') mit dem proximalen Ende des Schafts (120, 120') manuell lösbar ist, wobei das Montagewerkzeug (4) zur Zusammenwirkung mit einem der distalen Verbindungspartner (A, A') ausgebildet ist, um den Eingriff (A-A') des zusammenwirkenden distalen Verbindungspartners (A, A') zu lösen oder zu fügen; oder
- die Instrumentenkomponente mit dem integrierten Montagewerkzeug (4) die Werkzeugspitze (130, 130') ist, deren Eingriff (A-A') mit dem distalen Ende des Schafts (120, 120') manuell lösbar ist, wobei das Montagewerkzeug (4) zur Zusammenwirkung mit einem der proximalen Verbindungspartner (B, B') ausgebildet ist, um den Eingriff (B-B') des zusammenwirkenden proximalen Verbindungspartners (B,B') zu lösen oder zu fügen; oder
- die Instrumentenkomponente mit dem integrierten Montagewerkzeug (4) der Handgriff (110, 110') oder der Schaft (120, 120') oder die Werkzeugspitze (130, 130') ist, wobei das Montagewerkzeug (4) manuell lösbar mit der Instrumentenkomponente verbunden ist und zur Zusammenwirkung mit einem der distalen Verbindungspartner (A, A') und mit einem der proximalen Verbindungspartner (B, B') ausgebildet ist, um den Eingriff (A-A') des zusammenwirkenden distalen Verbindungspartners (A, A') und den Eingriff (B-B') des zusammenwirkenden proximalen Verbindungspartners (B, B') zu lösen oder zu fügen.

6. Chirurgisches Instrumentensystem (1) nach Anspruch 3,
**dadurch gekennzeichnet, dass**
das chirurgische Instrumentensystem (1) zum Zusammenbau von zumindest zwei zweiten chirurgischen Instrumenten (10") zumindest zwei zweite Sets aufweist, wobei jedem zweiten Set die weitere Instrumentenkomponente mit dem Montagewerkzeug (4) des anderen Sets zugeordnet ist, und wobei das Montagewerkzeug (4) nicht-lösbar an der weiteren Instrumentenkomponente vorliegt, und das Montagewerkzeug (4) des einen zweiten chirurgischen Instruments (10") zur Zusammenwirkung mit einem der distalen Verbindungspartner (A, A') und mit einem der proximalen Verbindungspartner (B, B') des jeweils anderen zweiten chirurgischen Instrument (10") ausgebildet ist, um den Eingriff (A-A') des zusammenwirkenden distalen Verbindungspartners (A, A') und den Eingriff (B-B') des zusammenwirkenden proximalen Verbindungspartners (B, B') des jeweils anderen zweiten chirurgischen Instrument (10") zu lösen oder zu fügen.

7. Chirurgisches Instrumentensystem (1) nach zumindest einem der Ansprüche 1 bis 6, **dadurch gekennzeichnet, dass**
das Montagewerkzeug (4) ein Eingriffsprofil (40) aufweist und der zusammenwirkende distale Verbindungspartner (A, A') oder proximale Verbindungspartner (B, B') ein Mitnahmeprofil (30) aufweist, das mit dem Eingriffsprofil (40) korrespondiert.

8. Chirurgisches Instrumentensystem (1) nach Anspruch 7,
**dadurch gekennzeichnet, dass**
- das Eingriffsprofil (40) des Montagewerkzeugs (4) in einem Funktionselement (112) der Instrumentenkomponente ausgebildet ist, das eine von der Montagefunktion abweichende Basisfunktion hat, oder
- das Montagewerkzeug (4) durch ein Werkzeugelement bereitgestellt wird, das das Eingriffsprofil (40) aufweist, wobei das Werkzeugelement schwenkbar oder lösbar an der Instrumentenkomponente angeordnet oder in einer Aussparung (113) der Instrumentenkomponente aufgenommen ist.

9. Chirurgisches Instrumentensystem (1) nach Anspruch 8,
**dadurch gekennzeichnet, dass**
- das Funktionselement (112) der Instrumentenkomponente mit der von der Montagefunktion abweichenden Basisfunktion ein Drehknopf (112 ) einer Rändelschraube ist, die zur manuellen Sicherung des proximalen Endes des Schafts (120, 120') im Handgriff (110, 110') angeordnet ist; oder
- die Aussparung (113) ein an das Montagewerkzeug (4) angepasstes Aufnahmefach (113) ist, das in einem Griffgehäuse (111) des Handgriffs (110, 110') ausgebildet ist.

10. Chirurgisches Instrumentensystem (1) nach zumindest einem der Ansprüche 7 bis 9, **dadurch gekennzeichnet, dass**
das Eingriffsprofil (40)
- einem Schraubenschlüssel-Werkzeugprofil aus der Gruppe, umfassend Außen- oder Innenmehrkant, Außen- oder Innenmehrrund oder Außen- oder Innenvielzahn, entspricht, oder
- einem Schraubenzieher-Werkzeugprofil für Schlitz- oder Kreuzschlitzschrauben entspricht, oder
- einer Zange entspricht, oder
- ein individuell gestaltetes Werkzeugprofil ist, das von dem Schraubenschlüssel-Werkzeugprofil, dem Schraubenzieher-Werkzeugprofil und der Zange abweicht.

11. Zerlegbares chirurgisches Instrument (10, 10', 10") mit einer Werkzeugspitze (130, 130'), einem Schaft (120, 120') und einem Handgriff (110, 110') als Instrumentenkomponenten, wobei die Werkzeugspitze (130, 130') und ein distales Ende des Schafts (120, 120') als distale Verbindungspartner (A, A') in lösbarem Eingriff (A-A') stehen, und ein proximales Ende des Schafts (120, 120') und der Handgriff (110, 110') als proximale Verbindungspartner (B, B') in lösbarem Eingriff (B-B') stehen,
**dadurch gekennzeichnet, dass**
das zerlegbare chirurgische Instrument (10, 10', 10") aus den Instrumentenkomponenten eines chirurgischen Instrumentensystems (1) nach zumindest einem der Ansprüche 1 bis 10 zusammengebaut ist.

12. Verfahren zur Montage und Demontage eines chirurgischen Instruments (10, 10', 10") nach Anspruch 11, das aus den Instrumentenkomponenten eines chirurgischen Instrumentensystems (1) nach zumindest einem der Ansprüche 1 bis 10 zusammenbaubar und zerlegbar ist,
**umfassend die Schritte**
- Bereitstellen der Instrumentenkomponente mit dem Montagewerkzeug (4), das zum Zusammenwirken mit zumindest einem der distalen Verbindungspartner (A, A') oder einem der proximalen Verbindungspartner (B, B') ausgebildet ist, und
- unter Verwendung des Montagewerkzeugs (4) bei der Montage des chirurgischen Instruments (10, 10', 10") Fügen zumindest des Eingriffs (A-A') der distalen Verbindungspartner (A, A') oder des Eingriffs (B-B') der proximalen Verbindungspartner (B, B') und bei der Demontage des chirurgischen Instruments (10, 10', 10") Lösen zumindest des Eingriffs (A-A') der distalen Verbindungspartner (A, A') oder des Eingriffs (B-B') der proximalen Verbindungspartner (B, B').

## Claims

1. A surgical instrument system (1) comprising the following instrument components:
- at least one tool tip (130, 130'),
- at least one shaft (120, 120') and
- at least one handle (110, 110'), wherein
the tool tip (130, 130') and a distal end of the shaft (120, 120') are in the form of distal mating elements (A, A') which can be connected in releasable engagement (A-A'), and a proximal end of the shaft (120, 120') and the handle (110, 110') are in the form of proximal mating elements (B, B') which can be connected in releasable engagement (B-B'),
**characterised in that**
at least one of the instrument components comprises an integrated assembly tool (4) which is designed to cooperate with at least one of the distal mating elements (A, A') or one of the proximal mating elements (B, B') to release or establish the engagement (A-A') of the cooperating distal mating element (A, A') and/or the engagement (B-B') of the cooperating proximal mating element (B, B').

2. The surgical instrument system (1) according to claim 1,
**characterised in that**
a first surgical instrument (10), which can be configured from at least one first set of the instrument components of the surgical instrument system (1), has the instrument component with the integrated assembly tool (4) as one of the distal mating elements (A, A') or the proximal mating elements (B, B'), wherein
- the engagement (A-A') or the engagement (B-B') of the instrument component, which has the integrated assembly tool (4), can be manually released from and established with the respective distal mating element (A, A') or proximal mating element (B, B') assigned to the instrument component with the integrated assembly tool (4),
or
- the assembly tool (4) is present on the instrument component in a manually releasable manner and is designed to cooperate with one of the distal mating elements (A, A') and one of the proximal mating elements (B, B') to release or establish the engagement (A-A') of the cooperating distal mating element (A, A') and the engagement (B-B') of the cooperating proximal mating element (B, B').

3. The surgical instrument system (1) according to claim 2,
**characterised in that**
the surgical instrument system (1) has at least one second set of instrument components from which a second surgical instrument (10', 10") can be configured, and at least one further instrument component which is a tool tip (130, 130') or a shaft (120, 120') or a handle (110, 110') and which has the integrated assembly tool (4), which is designed to cooperate with one of the distal mating elements (A, A') and with one of the proximal mating elements (B, B') of the second surgical instrument (10', 10") to release or establish the engagement (A-A') of the cooperating distal mating element (A, A') and the engagement (B-B') of the cooperating proximal mating element (B, B') of the second surgical instrument (10', 10").

4. The surgical instrument system (1) according to claim 3,
**characterised in that**
the first set for configuring the first surgical instrument (10) has the further instrument component with the integrated assembly tool (4), and the second surgical instrument (10') is a tool-free surgical instrument (10') that does not have an instrument component with the assembly tool (4).

5. The surgical instrument system (1) according to one of claims 2 to 4,
**characterised in that**
- the instrument component with the integrated assembly tool (4) is the handle (110, 110'), the engagement (B-B') of which with the proximal end of the shaft (120, 120') can be manually released, wherein the assembly tool (4) is designed to cooperate with one of the distal mating elements (A, A') to release or establish the engagement (A-A') of the cooperating distal mating element (A, A'); or
- the instrument component with the integrated assembly tool (4) is the tool tip (130, 130'), the engagement (A-A') of which with the distal end of the shaft (120, 120') can be manually released, wherein the assembly tool (4) is designed to cooperate with one of the proximal mating elements (B, B') to release or establish the engagement (B-B') of the cooperating proximal mating element (B, B'); or
- the instrument component with the integrated assembly tool (4) is the handle (110, 110') or the shaft (120, 120') or the tool tip (130, 130'), wherein the assembly tool (4) is connected to the instrument component in a manually releasable manner and is designed to cooperate with one of the distal mating elements (A, A') and with one of the proximal mating elements (B, B') to release or establish the engagement (A-A') of the cooperating distal mating element (A, A') and the engagement (B-B') of the cooperating proximal mating element (B, B').

6. The surgical instrument system (1) according to claim 3,
**characterised in that**
the surgical instrument system (1) for configuring at least two second surgical instruments (10") has at least two second sets, wherein each second set is assigned to the further instrument component with the assembly tool (4) of the other set, and wherein the assembly tool (4) is present on the further instrument component in a non-releasable manner, and the assembly tool (4) of the one second surgical instrument (10") is designed to cooperate with one of the distal mating elements (A, A') and with one of the proximal mating elements (B, B') of the respectively other second surgical instrument (10") to release or establish the engagement (A-A') of the cooperating distal mating element (A, A') and the engagement (B-B') of the cooperating proximal mating element (B, B') of the respectively other second surgical instrument (10").

7. The surgical instrument system (1) according to at least one of claims 1 to 6,
**characterised in that**
the assembly tool (4) has an engagement profile (40) and the cooperating distal mating element (A, A') or proximal mating element (B, B') has a drive profile (30) that corresponds to the engagement profile (40).

8. The surgical instrument system (1) according to claim 7,
**characterised in that**
- the engagement profile (40) of the assembly tool (4) is designed in a functional element (112) of the instrument component which has a basic function that differs from the assembly function, or
- the assembly tool (4) is provided by a tool element, which has the engagement profile (40), wherein the tool element is arranged on the instrument component in a pivotable or releasable manner or is received in a recess (113) of the instrument component.

9. The surgical instrument system (1) according to claim 8,
**characterised in that**
- the functional element (112) of the instrument component with the basic function that differs from the assembly function is a knob (112) on a knurled screw, which is arranged in the handle (110, 110') for manually securing the proximal end of the shaft (120, 120'); or
- the recess (113) is a receiving compartment (113) which is adapted to the assembly tool (4) and which is formed in a grip housing (111) of the handle (110, 110').

10. The surgical instrument system (1) according to at least one of claims 7 to 9, **characterised in that**
the engagement profile (40)
- corresponds to a wrench tool profile from the group comprising external or internal polygonal profile, external or internal multi-lobed profile, or external or internal multi-spline profile, or
- corresponds to a screwdriver tool profile for flat-head or Phillips screws, or
- corresponds to a forceps, or
- a customised tool profile that differs from the wrench tool profile, the screwdriver tool profile and the forceps.

11. A dismantleable surgical instrument (10, 10', 10") comprising a tool tip (130, 130'), a shaft (120, 120') and a handle (110, 110') as instrument components, wherein the tool tip (130, 130') and a distal end of the shaft (120, 120') are in releasable engagement (A-A') as distal mating elements (A, A'), and a proximal end of the shaft (120, 120') and the handle (110, 110') are in releasable engagement (B-B') as proximal mating elements (B, B'),
**characterised in that**
the dismantleable surgical instrument (10, 10', 10") is configured from the instrument components of a surgical instrument system (1) according to at least one of claims 1 to 10.

12. A method for assembling and disassembling a surgical instrument (10, 10', 10") according to claim 11, which can be configured and dismantled from the instrument components of a surgical instrument system (1) according to at least one of claims 1 to 10,
**comprising the steps**
- Providing the instrument component with the assembly tool (4), which is designed to cooperate with at least one of the distal mating elements (A, A') or one of the proximal mating elements (B, B'), and
- Using the assembly tool (4) during the assembly of the surgical instrument (10, 10', 10"), establishing at least the engagement (A-A') of the distal mating elements (A, A') or the engagement (B-B') of the proximal mating elements (B, B'), and during disassembly of the surgical instrument (10, 10', 10"), releasing at least the engagement (A-A') of the distal mating elements (A, A') or the engagement (B-B') of the proximal mating elements (B, B').

## Revendications

1. Système d'instruments chirurgicaux (1) comportant les composants d'instrument :
- au moins une pointe d'outil (130, 130'),
- au moins une tige (120, 120') et
- au moins une poignée (110, 110'), dans lequel
la pointe d'outil (130, 130') et une extrémité distale de la tige (120, 120') sont conçues comme des partenaires de liaison distaux (A, A'), qui peuvent être reliés par une mise en prise amovible (A-A'), et
une extrémité proximale de la tige (120, 120') et la poignée (110, 110') sont conçues comme des partenaires de liaison proximaux (B, B'), qui peuvent être reliés par une mise en prise amovible (B-B'),
**caractérisé en ce que**
au moins l'un des composants d'instrument présente un outil de montage (4) intégré, qui est conçu pour coopérer avec au moins l'un des partenaires de liaison distaux (A, A') ou l'un des partenaires de liaison proximaux (B, B'), afin de libérer ou de causer la mise en prise (A-A') du partenaire de liaison distal (A, A') coopérant et/ou la mise en prise (B-B') du partenaire de liaison proximal (B, B') coopérant.

2. Système d'instruments chirurgicaux (1) selon la revendication 1,
**caractérisé en ce que**
un premier instrument chirurgical (10), qui peut être assemblé à partir d'au moins un premier ensemble de composants d'instrument du système d'instruments chirurgicaux (1), présente le composant d'instrument comportant l'outil de montage (4) intégré en tant que l'un des partenaires de liaison distaux (A, A') ou des partenaires de liaison proximaux (B, B'), dans lequel
- la mise en prise (A-A') ou la mise en prise (B-B') du composant d'instrument, qui présente l'outil de montage (4) intégré, peut être libérée et causée manuellement avec le partenaire de liaison distal (A, A') ou partenaire de liaison proximal (B, B') respectif, qui est associé au composant d'instrument comportant l'outil de montage (4) intégré, ou
- l'outil de montage (4) se trouve de manière amovible manuellement sur le composant d'instrument et est conçu pour coopérer avec l'un des partenaires de liaison distaux (A, A') et l'un des partenaires de liaison proximaux (B, B') afin de libérer ou de causer la mise en prise (A-A') du partenaire de liaison distal (A, A') coopérant et la mise en prise (B-B') du partenaire de liaison proximal (B, B') coopérant.

3. Système d'instruments chirurgicaux (1) selon la revendication 2,
**caractérisé en ce que**
le système d'instruments chirurgicaux (1) présente au moins un second ensemble de composants d'instrument, à partir desquels un second instrument chirurgical (10', 10") peut être assemblé, ainsi qu'au moins un composant d'instrument supplémentaire, qui est une pointe d'outil (130, 130') ou une tige (120, 120') ou une poignée (110, 110') et qui présente l'outil de montage (4) intégré, qui est conçu pour coopérer avec l'un des partenaires de liaison distaux (A, A') et avec l'un des partenaires de liaison proximaux (B, B') du second instrument chirurgical (10', 10") afin de libérer ou de causer la mise en prise (A-A') du partenaire de liaison distal (A, A') coopérant et la mise en prise (B-B') du partenaire de liaison proximal (B, B') coopérant du second instrument chirurgical (10', 10").

4. Système d'instruments chirurgicaux (1) selon la revendication 3,
**caractérisé en ce que**
le premier ensemble pour assembler le premier instrument chirurgical (10) présente le composant d'instrument supplémentaire comportant l'outil de montage (4) intégré, et le second instrument chirurgical (10') est un instrument chirurgical sans outil (10'), qui ne présente pas de composant d'instrument comportant l'outil de montage (4).

5. Système d'instruments chirurgicaux (1) selon l'une des revendications 2 à 4, **caractérisé en ce que**
- le composant d'instrument comportant l'outil de montage (4) intégré est la poignée (110, 110'), dont la mise en prise (B-B') avec l'extrémité proximale de la tige (120, 120') peut être libérée manuellement, dans lequel l'outil de montage (4) est conçu pour coopérer avec l'un des partenaires de liaison distaux (A, A') afin de libérer ou de causer la mise en prise (A-A') de l'élément de liaison distal (A, A') coopérant ; ou
- le composant d'instrument comportant l'outil de montage (4) intégré est la pointe d'outil (130, 130'), dont la mise en prise (A-A') avec l'extrémité distale de la tige (120, 120') peut être libérée manuellement, dans lequel l'outil de montage (4) est conçu pour coopérer avec l'un des partenaires de liaison proximaux (B, B') afin de libérer ou de causer la !mise en prise (B-B') de l'élément de liaison proximal (B, B') coopérant ; ou
- le composant d'instrument comportant l'outil de montage (4) intégré, est la poignée (110, 110') ou la tige (120, 120') ou la pointe d'outil (130, 130'), dans lequel l'outil de montage (4) est relié de manière amovible manuellement au composant d'instrument et est conçu pour coopérer avec l'un des partenaires de liaison distaux (A, A') et avec l'un des partenaires de liaison proximaux (B, B') afin de libérer ou de causer la mise en prise (A-A') du partenaire de liaison distal (A, A') coopérant et la mise en prise (B-B') du partenaire de liaison proximal (B, B') coopérant.

6. Système d'instruments chirurgicaux (1) selon la revendication 3,
**caractérisé en ce que**
le système d'instruments chirurgicaux (1) pour assembler au moins deux seconds instruments chirurgicaux (10") présente au moins deux seconds ensembles, dans lequel chaque second ensemble est associé au composant d'instrument supplémentaire comportant l'outil de montage (4) de l'autre ensemble, et dans lequel l'outil de montage (4) se trouve de manière inamovible sur le composant d'instrument supplémentaire, et l'outil de montage (4) de l'un des seconds instruments chirurgicaux (10") est conçu pour coopérer avec l'un des partenaires de liaison distaux (A, A') et avec l'un des partenaires de liaison proximaux (B, B') de l'autre second instrument chirurgical (10") respectif, afin de libérer ou de causer la mise en prise (A-A') du partenaire de liaison distal (A, A') coopérant et la mise en prise (B-B') du partenaire de liaison proximal (B, B') coopérant de l'autre second instrument chirurgical (10") respectif.

7. Système d'instruments chirurgicaux (1) selon au moins l'une des revendications 1 à 6, **caractérisé en ce que**
l'outil de montage (4) présente un profil de mise en prise (40) et le partenaire de liaison distal (A, A') ou partenaire de liaison proximal (B, B') coopérant présente un profil d'entraînement (30), qui correspond au profil de mise en prise (40).

8. Système d'instruments chirurgicaux (1) selon la revendication 7,
**caractérisé en ce que**
- le profil de mise en prise (40) de l'outil de montage (4) est conçu dans un élément fonctionnel (112) du composant d'instrument, qui a une fonction de base différente de la fonction de montage, ou
- l'outil de montage (4) est fourni par un élément d'outil, qui présente le profil de mise en prise (40), dans lequel l'élément d'outil est agencé de manière pivotante ou amovible sur le composant d'instrument ou logé dans un évidement (113) du composant d'instrument.

9. Système d'instruments chirurgicaux (1) selon la revendication 8,
**caractérisé en ce que**
- l'élément fonctionnel (112) du composant d'instrument comportant la fonction de base différant de la fonction de montage, est un bouton rotatif (112) d'une vis moletée, qui est agencé dans la poignée (110, 110') afin de fixer manuellement l'extrémité proximale de la tige (120, 120') ; ou
- l'évidement (113) est un compartiment (113) adapté à l'outil de montage (4), qui est conçu dans un boîtier de poignée (111) de la poignée (110, 110').

10. Système d'instruments chirurgicaux (1) selon au moins l'une des revendications 7 à 9, **caractérisé en ce que**
le profil de mise en prise (40)
- correspond à un profil d'outil de clé à molette du groupe, comprenant les profils polygonaux extérieurs ou intérieurs, les profils circulaires extérieurs ou intérieurs ou les profils à dents multiples extérieurs ou intérieurs, ou
- correspond à un profil d'outil de tournevis pour vis à fente ou à tête cruciforme, ou
- correspond à une pince, ou
- est un profil d'outil construit individuellement, qui s'écarte du profil d'outil de clé à molette, du profil d'outil de tournevis et de la pince.

11. Instrument chirurgical déconstructible (10, 10', 10") comportant une pointe d'outil (130, 130'), une tige (120, 120') et une poignée (110, 110') en tant que composants d'instrument, dans lequel la pointe d'outil (130, 130') et une extrémité distale de la tige (120, 120') sont en prise amovible (A-A') en tant que partenaires de liaison distaux (A, A'), et une extrémité proximale de la tige (120, 120') et la poignée (110, 110') sont en prise amovible (B-B') en tant que partenaires de liaison proximaux (B, B'),
**caractérisé en ce que**
l'instrument chirurgical déconstructible (10, 10', 10") est assemblé à partir des composants d'instrument d'un système d'instruments chirurgicaux (1) selon au moins l'une des revendications 1 à 10.

12. Procédé de montage et de démontage d'un instrument chirurgical (10, 10', 10") selon la revendication 11, qui peut être assemblé et déconstruit à partir des composants d'instrument d'un système d'instruments chirurgicaux (1) selon au moins l'une des revendications 1 à 10,
**comprenant les étapes**
- fourniture du composant d'instrument comportant l'outil de montage (4), qui est conçu pour coopérer avec au moins l'un des partenaires de liaison distaux (A, A') ou l'un des partenaires de liaison proximaux (B, B'), et
- lors de l'utilisation de l'outil de montage (4) lors du montage de l'instrument chirurgical (10, 10', 10") le fait de causer au moins la mise en prise (A-A') des partenaires de liaison distaux (A, A') ou la mise en prise (B-B') des partenaires de liaison proximaux (B, B') et, lors du démontage de l'instrument chirurgical (10, 10', 10") la libération au moins de la mise en prise (A-A') des partenaires de liaison distaux (A, A') ou la mise en prise (B-B') des partenaires de liaison proximaux (B, B').
